# EUROPEAN PATENT APPLICATION

(11) **EP 2 601 947 A1**
(43) Date of publication of application: **12.06.2013**
(21) Application number: 11192001.3
(22) Date of filing: 05.12.2011
(51) Int. Cl.: A61K 31/341, A61K 31/4164, A61K 31/43, A61K 31/4439, A61K 31/7048, A61K 9/24, A61K 45/06, A61P 1/04, A61P 31/04

(54) **Fixed-dose combination for treatment of helicobacter pylori associated diseases**

(71) Applicant: Al-Mehdar, Abo Bakr Mohammed Ali, Riyadh (SA)
(72) Inventor: Al-Mehdar, Abo Bakr Mohammed Ali, Riyadh (SA)
(74) Representative: Straube, Urs Norman

(57) **Abstract**

The present invention relates to an oral pharmaceutical composition for treatment of helicobacter pylori associated diseases like ulcers comprising an acid blocker together with at least one antibiotic. For better patient compliance a fixed-dose combination is proposed.

## Description

The present invention relates an oral pharmaceutical composition for treatment of helicobacter pylori associated diseases like ulcers comprising an acid blocker together with at least one antibiotic.

The relationship between gastrointestinal ulcers and infection with Helicobacter pylori proposed in 1983 by Warren (Warren J R Lancet 1983; 1. 1273) is well established. A number of different therapies have been proposed for treatment of H. pylori infections. Most of these therapies comprise different combinations of antibacterial compounds. Some of these therapies also comprise a bismuth compound; see for instance WO 89/03219 (Borody). Other combination therapies comprise a proton pump inhibitor and one or more antibacterial compounds, for instance a combined regimen of omeprazole and amoxicillin which has been approved by regulatory authorities in for example Great Britain and Sweden for the treatment of H. pylori infections. Different triple therapies, for example omeprazole, clarithromycin and amoxicillin or other antibacterial substances, have been reported at the 10^{th} World Congresses of Gastroenterology in October 1994. Some published patent applications in this field are for instance: WO 93/00327, Astra Aktiebolag, which discloses the combination of a substance with inhibiting effect on the gastric acid secretion which increases the intra-gastric pH and an antibacterial compound and WO 92/03135, Smithkline & French Laboratories, which discloses a combination of a benzimidazole and an anti-Helicobacter agent, i.e. for instance pantoprazole in combination with amoxicillin and/or metronidazole.

Initially, treatment was based on administering antacids, such as magnesium or aluminum compounds, calcium carbonates, alkaline bismuth salts, e.g. bismuth aluminates, colloidal bismuth salts. A high relapse rate of over 80% and side effects, such as the rebound effect of acid secretion, deposits of aluminum and bismuth salts in the tissue to bismuth nephropathy, and bismuth encephalopathy forced the medical field to pursue new paths.

After proof has been secured of H. pylori following gastroscopy and removal of the mucosa of the stomach from the antrum or corpus, eradication of the germ through combination therapy is necessary. Serious H. pylori related forms of gastritis and underdevelopment should be treated in accordance with cancer prophylaxis-stomach carcinoma and lymphoma (MALT). Treatment turned out to be very difficult. Due to a high relapse rate, the mono- and dual-therapy was insufficient. For mono-therapy with bismuth compounds, and for dual-therapy with amoxicillin and omeprazole, the eradication rate was only between 35 and 60%. Through the combination of bismuth, amoxicillin and metronidazole, healing was achieved for the first time in 85-95% of the cases after a 4-week treatment. The toxicity of this 3-fold combination, however, has not yet been clarified, especially when taking the long treatment time of 4 weeks into consideration.

Based on their randomized, double blind, placebo-controlled trial, Vaira, Zullo, Vakil et al. conclude that sequential therapy with a proton pump inhibitor (abbreviated as "PPI") for 10 days, amoxicillin for 5 days followed by both clarithromycin and tinidazole for 5 additional days is more effective than conventional triple therapy for Helicobacter Pylori eradication (pantoprazole, amoxicillin and clarithromycin for 10 days) in eradicating H. pylori among symptomatic patients.

It is well known that proton pump inhibitors are susceptible to degradation/ transformation in acid reacting and neutral media. In respect to stability, it is obvious that one of the active substances being a proton pump inhibitor must be protected from contact with acidic gastric juice, for example by an enteric coating layer. There are different enteric coating layered preparations of omeprazole as well as other proton pump inhibitors described in the prior art, see for example U.S. Pat. No. 4,786,505 (A B Hassle).

The triple therapy is a 7-day treatment with a proton pump inhibitor (abbreviated as "PPI") as well as two antibiotics. According to the "Italian Triple Therapy" these are clarithromycin and metronidazole and according to the "French Triple Therapy" these are clarithromycin and amoxicillin. The medication must be taken twice daily each in standard dosage. This means that the patient must take many tablets a day. Also, there are side effects of the antibiotics, especially diarrhea, taste changes, etc. An antibiotics resistance development is also disadvantageous. H. pylori resistance to metronidazole, clarithromycin and amoxicillin has already been found. In the dual, triple and sequential therapy that are proposed to treat gastric ulcer associated with H. Pylori infection, each single active substance is administered separately in different dosage forms each of them comprises only one single active substance.

However it is well known that patient compliance is a main factor in receiving a good result in medical treatments especially in the treatment of H. pylori infections. Administration of two, three or even more different tablets or capsules to the patient is not convenient or satisfactory to achieve the most optimal results.

Therefore it is an objective of the present invention to propose an oral formulation for treatment of helicobacter pylori associated diseases comprising an acid blocker together with at least one antibiotic, which has a better patient compliance.

This objective is achieved by an orally administered formulation for the treatment of gastric and duodenal ulcers associated with Helicobacter pylori infections in a form of a fixed-dose combination. Instead of an oral administration of two, three or even more different dosage forms administration of only one fixed-dose combination is sufficient. The formulation optionally comprises suitable, pharmaceutically acceptable excipients.

The fixed-dose formulation comprises at least one conventional antibiotic. However H. pylori is resistant to wide variety of conventionally used antibiotics including amoxicillin, clarithromycin, metronidazole, tinidazole and other wide range of antibiotics. It has been reported that cure rate is reduced from over 90 % to less than 60 % with increasing incidence of infection relapse. Therefore, an urgent task to be solved is reducing bacterial resistance and improving cure rates. To provide an enhanced Helicobacter pylori eradication, one or more conventional antibiotics are combined with an amount of an antimicrobial medicament selected from the group consisting of taurolidine, taurultam or a combination thereof. Taurolidine ([bis (1,1-dioxoperhydro-1,2,4-thiadiazinyl-4)-methane) is a drug with antimicrobial and anti-lipopolysaccharide properties. Derived from the amino acid taurine, its immune modulatory action is reported to be mediated with priming and activation of macrophages and polymorphonuclear leukocytes. Taurolidine has been used to treat patients with peritonitis and as an agent in patients with Systemic inflammatory response syndrome. Additionally, taurolidine demonstrates some anti-tumor properties, with positive results seen in early-stage clinical investigations using the drug to treat gastrointestinal malignancies and tumors of the central nervous system. Taurolidine has been investigated for the prevention of central venous catheter-related infections; while there is positive in vivo and in vitro evidence supporting such an application, the current evidence is "insufficient to warrant routine use of taurolidine". Taurolidine and/or taurultam reduce significantly Helicobacter pylori resistance to conventional antibiotics and to reduce the risk for developing bacterial resistance. Taurolidine and taurultam are cell-wall cross-linking compounds effective in the treatment of gastric and duodenal ulcer initiated by H. pylori. Taurolidine is a unique antimicrobial agent having an exceptionally broad spectrum of antimicrobial and antibacterial activity including activity against gram positive and gram negative, aerobic, and anaerobic bacteria. Resistance has not been observed either in vivo or in vitro. The compounds taurolidine and taurultam are disclosed in U.S. Patent No. 5,210,083. Taurolidine's mechanism of action unlike that of known antibiotics is based on a chemical reaction. While not being bound by any theory, during the metabolism of taurolidine to taurinamide and ultimately taurine and water, methylol groups are liberated which chemically react with the mureins in the bacterial cells walls this results in the denaturing of the complex polysaccharide and liposaccharide components of the bacterial cell wall as well as changing the double stranded DNA of the plasmid to a denatured or single stranded DNA. taurolidine has been shown to be safe and well tolerated at systemic doses exceeding 40 g/day and cumulative doses up to and exceeding 300 g. taurolidine is formulated as tablets, capsules and liquids either alone or in combination with antacid. It is recommended to be concomitantly administered with a PPI and other antibacterial compounds for synergistic effect. However fixed unit dosage forms of taurolidine either in combination with a PPI and/or other antibacterial compounds is not reported.

The fixed-dose formulation may additionally comprise zinc amino chelate for strengthening the stomach mucosa, sticking to the stomach wall and acting as a buffer to gastric acid, serving as an antioxidant, controlling the inflammatory response to stomach injury, and inhibiting the growth of H. pylori bacteria probably by strengthening the stomach mucosa and making it less susceptible to a bacterial infection.

The fixed-dose formulation may comprise nitroiridazole antibiotics, tetracycline, penicillins, cephalosporins, carbopenems, amino glycosides, macrolide antibiotics, lincosamide antibiotics, 4-quinolones, rifamycins and nitrofurantoin. In the following examples of such antibacterial compounds are listed: ampicillin, amoxicillin, benzyl penicillin, phenoxymethylpeniclllin, bacampicillin, pivampicillin, carbenicillin, cloxacillin, cyclacillin, dicloxacillin, methicillin, oxacillin, piperacillin, ticarcillin, flucloxacillin, cefuroxime, cefetamet, cefetrame, cefixime, cefoxitin, ceftazidime, ceftizoxime, latamoxef, cefoperazone, ceftriaxone, cefsulodin, cefotaxime, cephalexin, cefaclor, cefadroxil, cefalothin, cefazolin, cefpodoxime, ceftibuten, aztreonam, tigemonam, erythromycin, dirithromycin, roxithromycin, azithromycin, clarithromycin, clindamycin, paldimycin, lincomycin, vancomycin, spectinomycin, tobramycin, paromomycin, metronidazole, tinidazole, ornidazole, amifloxacin, cinoxacin, ciprofloxacin, difloxacin, enoxacin, fleroxacin, norfloxacin, ofloxacin, temafloxacin, doxycycline, minocycline, tetracycline, chlortetracycline, oxytetracycline, methacycline, rolitetracyclin, nitrofurantoin, nalidixic acid, gentamicin, rifampicin, amikacin, netilmicin, imipenem, cilastatin, chloramphenicol, furazolidone, nifuroxazide, sulfadiazin, sulfametoxazol, bismuth subsalicylate, colloidal bismuth subcitrate, gramicidin, mecillinam, cloxiquine, chlorhexidine, dichlorobenzylalcohol, methyl-2-pentylphenol. The active antibacterial agents could be in standard forms or used as salts, hydrates, esters etc. However amoxicillin or a combination of tinidazole and clarithromycin is preferred.

The acid blocker of the fixed-dose formulation is selected from the group of H2 receptor antagonists, ranitidine is preferred.

Alternatively, the acid blocker is selected from the group of proton pump inhibitors, which may be used in neutral form or in the form of an alkaline salt, such as for instance the Mg², Ca², Na.sup., K.sup. or Li.sup. The compounds may be used in racemic form or in the form of a substantially pure enantiomer thereof, or alkaline salts of the single enantiomer. Suitable proton pump inhibitors are for example disclosed in EP-A1-0005129, EP-A1-174 726, EP-A1-166 287, GB 2 163 747 and WO90/06925, WO91/19711, WO91/19712, and further especially suitable compounds are described in WO95/01977 and WO94/27988. Omeprazole is preferred.

The fixed-dose formulation may comprise a suitable pharmaceutical alkanizer, preferably tromethamole. The tromethamole stabilizes the PPls and other combined active ingredients against gastric acid degradation. It raises the stomach pH above the pKa of the acid susceptible PPls and hence increases their resistance against gastric acid degradation. Tromethamole is the preferred alkalinizer since it can be incorporated in considerably small quantities in the formulation. It has been reported in previous patents that PPls e.g. omeprazole are prepared in the form of immediate release dosage form in combination with sodium bicarbonate and/or Magnesium hydroxide. Such said alkalinizers have to be incorporated in considerably large amounts which are quite bulky to fit in multicomponent fixed dosage forms. In addition to that cautions are to be considered when giving sodium salts to hypertensive patients. Tromethamole buffers gastric acidity successfully in a much smaller dose ranging between 50 to 300 mg per dose.

The fixed-dose composition may be an instant release formulation. This avoids known problems with fixed unit dosage forms comprising rather high amount of active substances. It is well known that preparation of fixed unit tableted dosage form raises specific problems when enteric coating layered pellets containing acid susceptible proton pump inhibitors as active substance are compressed into tablets. Since the enteric coating layer often does not withstand the compression of the pellets into tablets, the susceptible active substance will be destroyed upon administration by penetrating acidic gastric juice and the acid resistance of the enteric coating layer of the pellets will not be sufficient in the tablet after compression. The instant release formulation releases their active ingredients in the stomach for immediate effects and is devoid of the lengthy enteric coating procedures that produces dosage forms with delayed action.

The fixed-dose composition may be formulated as tablets, capsules, effervescent granules or powder, effervescent tablets, liquids, dispersible and/or soluble tablets, dispersible powder, suspensions, sachets, premix syrups, jellies or glycerites, preferably as compressed tablets. Dispersible formulations are suitable for children and patients with swallowing disorders. Dispersible powders and tablets are also suitable for naso-gastric administration.

Alternatively, the fixed-dose composition may be formulated as a biphasic release form, preferably as a biphasic tablet with an inner slow release layer and an outer fast release layer.

The biphasic release of the ingredients can be achieved, if the inner layer is an enteric coated core. Especially proton pump inhibitors like omeprazole are acid-unstable. The enteric coating prevents release of the active ingredient or the active ingredients inside the core before they reach the small intestine. The delayed release avoids the stomach's acidic exposure and delivers them to the basic pH environment of the intestine. The core is coated by compression over the whole surface with a fast-disintegrating layer, preferably formulated as a compressed tablet. The outer layer, respectively the compressed tablet, comprises the antibiotic or the combination of antibiotics.

It is preferred that the slow release layer comprises an amount of the antibiotic or an amount of the combined antibiotics and the fast release layer comprises the remaining amount of the antibiotic or the remaining amount of the combined antibiotics.

The preferred antibiotic is amoxicillin.

The preferred combination of antibiotics comprises tinidazole and clarithromycin.

It is preferred that the outer layer also comprises taurolidine.

In the following preferred embodiments of the invention are described.

The table shows an overview of formulations with their active ingredients. The listed embodiments 1 to 41 are described in detail below the table.

| | Ingredient | Ingredient/s | Ingredient | Formulation |
|---|---|---|---|---|
| 1 | omeprazole | amoxicillin | | tablets |
| 2 | omeprazole | tinidazole/clarithromycin | | tablets |
| 3 | omeprazole | amoxicillin | taurolidine | tablets |
| 4 | omeprazole | tinidazole/clarithromycin | taurolidine | tablets |
| 5 | omeprazole | amoxicillin | zinc am. acid chelate | tablets |
| 6 | omeprazole | tinidazole/clarithromycin | zinc am. acid chelate | tablets |
| 7 | ranitidine | amoxicillin | | tablets |
| 8 | ranitidine | tinidazole/clarithromycin | | tablets |
| 9 | ranitidine | amoxicillin | taurolidine | tablets |
| 10 | ranitidine | tinidazole/clarithromycin | taurolidine | tablets |
| 11 | ranitidine | amoxicillin | zinc am. acid chelate | tablets |
| 12 | ranitidine | tinidazole/clarithromycin | zinc am. acid chelate | tablets |
| 13 | omeprazole | amoxicillin | | sachets |
| 14 | omeprazole | tinidazole/clarithromycin | | sachets |
| 15 | omeprazole | amoxicillin | taurolidine | sachets |
| 16 | omeprazole | tinidazole/clarithromycin | taurolidine | sachets |
| 17 | omeprazole | amoxicillin | zinc am. acid chelate | sachets |
| 18 | omeprazole | tinidazole/clarithromycin | zinc am. acid chelate | sachets |
| 19 | ranitidine | amoxicillin | | sachets |
| 20 | ranitidine | tinidazole/clarithromycin | | sachets |
| 21 | ranitidine | amoxicillin | taurolidine | sachets |
| 22 | ranitidine | tinidazole/clarithromycin | taurolidine | sachets |
| 23 | ranitidine | amoxicillin | zinc am. acid chelate | sachets |
| 24 | ranitidine | tinidazole/clarithromycin | zinc am. acid chelate | sachets |
| 25 | omeprazole | amoxicillin | | powder |
| 26 | omeprazole | tinidazole/clarithromycin | | powder |
| 27 | omeprazole | amoxicillin | taurolidine | powder |
| 28 | omeprazole | tinidazole/clarithromycin | taurolidine | powder |
| 29 | omeprazole | amoxicillin | zinc amino acid chelate | powder |
| 30 | omeprazole | tinidazole/clarithromycin | zinc am. acid chelate | powder |
| 31 | ranitidine | amoxicillin | | powder |
| 32 | ranitidine | tinidazole/clarithromycin | | powder |
| 33 | ranitidine | amoxicillin | taurolidine | powder |
| 34 | ranitidine | tinidazole/clarithromycin | taurolidine | powder |
| 35 | ranitidine | amoxicillin | zinc am. acid chelate | powder |
| 36 | ranitidine | tinidazole/clarithromycin | zinc am. acid chelate | powder |
| 37 | omeprazole | | taurolidine | packets |
| 38 | omeprazole | amoxicillin | + - taurolidine | biphasic tablets |
| 39 | omeprazole | tinidazole/clarithromycin | + - taurolidine | biphasic tablets |
| 40 | omeprazole | amoxicillin | zinc am. acid chelate | biphasic tablets |
| 41 | omeprazole | tinidazole/clarithromycin | zinc am. acid chelate | biphasic tablets |

In embodiment 1 compressed tablets in multiple component fixed unit dosage forms are made from omeprazole in a dose from 10 to 60 mg, preferably from 20 to 30 mg and more preferably from 20-40 mg, amoxicillin in a dose from 500 to 800 mg, preferably from 800 to 1000 mg and more preferably from 500 to 1000 mg. The tablets also contain tromethamole used as a buffering alkalinizer to enhance the stability of omeprazole in gastric acidity in a dose from 50-200 mg, preferably from 70- 200 mg and more preferably from 50-300 mg. Additional variety of pharmaceutical excipients including starch glycolate at between 50-135 mg, Talc at between 50 - 100 mg, Arosil 200 between 1.5-4.5 mg, sodium lauryl sulfate between 1-4 mg, sodium stearyl fumarate between 10-20 mg, magnesium stearate between 4.5-13.5 mg, in addition to other required pharmaceutical additives.

In embodiment 2 the amoxicillin in embodiment 1 is substituted with a combination containing both tinidazole and clarithromycin with the rest of active ingredients (omeprazole) and the inactive ingredients (tromethamole and excipients) are remained unchanged. tinidazole is included in the formulation at a dose of between 100-200 mg, preferably between 300-400 mg and more preferably between 250-500 mg. clarithromycin is included at a dose between 200-300 mg, preferably between 250-400 mg and more preferably from 250-500 mg.

The compressed tablets from embodiment 1) and embodiment 2) are indicated in sequential therapy program for the treatment of gastric and duodenal ulcers associated with H. Pylori infections. The tablets from embodiment 1) are to be administered twice daily for 5 days, followed by the compressed tablets from embodiment 2) to be taken as one tablet twice daily for 5 more consecutive days.

In embodiment 3 compressed tablets in fixed multiple component dosage forms are made from omeprazole in a dose from 10 to 60 mg, preferably from 20 to 30 mg and more preferably from 20-40 mg, amoxicillin at a dose from 500 to 800 mg, preferably from 800 to 1000 mg and more preferably from 500 to 1000 mg. The tablet also contains tromethamole used as a buffering alkalinizer at a dose from 50-200 mg, preferably from 70- 200 mg and more preferably from 50-300 mg. Additionally taurolidine/taurultam is included in the formula in order to synergies the activity of the amoxicillin and to reduce bacterial resistance due to it bacterial cell cross-linking effect. Taurolidine/taurultam is combined in a dose between 200 - 400 mg; Additionally taurolidine is included in the formula to reducing bacterial resistance against clarithromycin and tinidazole. taurolidine is included in doses between 100 - 200 mg, preferably from 200 - 400 mg and more preferably at between 250-500 mg. Additional variety of pharmaceutical excipients including Starch glycolate at between 50-135 mg, Talc at between 50 - 100 mg, Arosil 200 between 1.5-4.5 mg, sodium lauryl sulfate between 1-4 mg, sodium stearyl fumarate between 10-20 mg, Magnesium Stearate between 4.5-13.5 mg, in addition to other required pharmaceutical additives.

In embodiment 4 the amoxicillin in embodiment 3) is substituted with a combination containing both tinidazole and clarithromycin with the rest of the active ingredient (omeprazole and taurolidine) and the inactive ingredients (tromethamole and the other excipients) in embodiment 3) are remained unchanged. tinidazole is included in the formulation at a dose of between 100-200 mg, preferably between 300-400 mg and more preferably between 250-500 mg. clarithromycin is included at a dose between 200-300 mg, preferably between 250-400 mg and more preferably from 250-500 mg.

The compressed tablets from embodiment 3) and embodiment 4) are indicated in sequential therapy program for the treatment of gastric and duodenal ulcers associated with H. Pylori infections. The tablets from embodiment 3) are to be administered twice daily for 5 days, followed by the compressed tablets from embodiment 4) to be taken as one tablet twice daily for 5 more consecutive days.

In embodiment 5 the taurolidine included in embodiment (3) is substituted with zinc amino acid chelate in a dose between 10 mg to 20 mg/tablet preferably between 25 mg to 30 mg/tablet and most preferably between 40 mg to 100 mg the rest of other active ingredients (omeprazole and amoxicillin) and inactive ingredients (tromethamole and excipients) are remained unchanged. The prepared tablets are film coated.

In embodiment 6 the taurolidine included in embodiment (4) is substituted with zinc amino acid chelate in a dose between 10 mg to 20 mg/tablet preferably between 25 mg to 30 mg/tablet and most preferably between 40 mg to 100 mg the rest of other active ingredients (omeprazole plus tinidazole plus clarithromycin) and inactive ingredients (tromethamole and excipients) are remained unchanged. The prepared tablets are film coated.

The tablets from embodiment 5) and embodiment 6) are indicated in sequential therapy program for the treatment of gastric and duodenal ulcers associated with H. Pylori infections. The tablets from embodiment 5) are to be administered twice daily for 5 days, followed by the tablets from embodiment 6) to be taken as one tablet twice daily for 5 more consecutive days.

In embodiment 7 compressed tablets (in fixed multiple component dosage forms) are made from ranitidine (H2 receptor antagonist) in a dose from 75 - 150 mg, preferably from 100-300 mg, more preferably from 150-300 mg in combination with amoxicillin in a dose from 500 to 800 mg, preferably from 800 to 1000 mg and more preferably from 500 to 1000 mg. The tablets also contain variety of pharmaceutical excipients including Starch glycolate at between 50-135 mg, Talc at between 50 - 100 mg, Arosil 200 between 1.5-4.5 mg, sodium lauryl sulfate between 1-4 mg, sodium stearyl fumarate between 10-20 mg, magnesium stearate between 4.5-13.5 mg, in addition to other required pharmaceutical additives.

In embodiment 8 the amoxicillin in embodiment 7) is substituted with a combination containing both tinidazole and clarithromycin with the rest of active ingredients (ranitidine) and the inactive ingredients (Tablet excipients) are remained unchanged. tinidazole is included in the formulation at a dose of between 100-200 mg, preferably between 300-400 mg and more preferably between 250-500 mg. clarithromycin is included at a dose between 200-300 mg, preferably between 250-400 mg and more preferably from 250-500 mg.

The compressed tablets from embodiment 7) and embodiment 8) are indicated in sequential therapy program for the treatment of gastric and duodenal ulcers associated with H. Pylori infections. The tablets from embodiment 7) are to be administered twice daily for 5 days, followed by the compressed tablets from embodiment 8) to be taken as one tablet twice daily for 5 more consecutive days.

In embodiment 9 compressed tablets (in fixed multiple components dosage forms) comprising ranitidine (H2 receptor antagonist) in a dose from 75 - 150 mg, preferably from 100-300 mg, more preferably from 150-300 mg in combination with amoxicillin in a dose from 500 to 800 mg, preferably from 800 to 1000 mg and more preferably from 500 to 1000 mg. taurolidine is additionally included in the formula to reducing bacterial resistance against amoxicillin. Taurolidine is included in doses between 100 - 200 mg, preferably from 200 - 400 mg and more preferably at between 250-500 mg. The tablets also contain variety of pharmaceutical excipients including Starch glycolate at between 50-135 mg, Talc at between 50 - 100 mg, Arosil 200 between 1.5-4.5 mg, sodium lauryl sulfate between 1-4 mg, sodium stearyl fumarate between 10-20 mg, Magnesium Stearate between 4.5-13.5 mg, in addition to other required pharmaceutical additives. The prepared tablets are film coated.

In embodiment 10 amoxicillin included in embodiment 9) is substituted with a combination from tinidazole and clarithromycin with the rest of other active ingredients (ranitidine and taurolidine) and inactive ingredients (the excipients) are remained unchanged. tinidazole is included in the formulation at a dose of between 100-200 mg, preferably between 300-400 mg and more preferably between 250-500 mg. clarithromycin is included in a dose between 200-300 mg, preferably between 250-400 mg and more preferably from 250-500 mg. The prepared tablets are film coated.

The tablets from embodiment 9) and embodiment 10) are to be indicated in sequential therapy program for the treatment of gastric and duodenal ulcers associated with H. Pylori infections. The tablets from embodiment 9) are to be administered twice daily for 5 days, followed by the tablets from embodiment 10) to be taken as one tablet twice daily for 5 more consecutive days.

In embodiment 11 the taurolidine included in embodiment 9) is substituted with zinc amino acid chelate in a dose between 10 mg to 20 mg/tablet preferably between 25 mg to 30 mg/tablet and most preferably between 30 mg to 100 mg the rest of other active ingredients (ranitidine plus amoxicillin) and inactive ingredients (excipients) are remained unchanged. The prepared tablets are film coated.

In embodiment 12 the taurolidine included in embodiment 10) is substituted with zinc amino acid chelate in a dose between 10 mg to 20 mg/tablet preferably between 25 mg to 30 mg/tablet and most preferably between 30 mg to 100 mg the rest of other active ingredients (ranitidine plus tinidazole plus clarithromycin) and inactive ingredients (excipients) are remained unchanged. The prepared tablets are film coated.

The tablets from embodiment 11) and embodiment 12) are indicated in sequential therapy program for the treatment of gastric and duodenal ulcers associated with H. Pylori infections. The tablets from embodiment 11) are to be administered twice daily for 5 days, followed by the tablets from embodiment 12) to be taken as one tablet twice daily for 5 more consecutive days.

In embodiment 13 the multiple components fixed dosage forms are made in single dose packets (sachets) comprising omeprazole, amoxicillin and tromethamole. Omeprazole is included in a dose from 10 to 60 mg, preferably from 20 to 30 mg and more preferably from 20-40 mg, amoxicillin in a dose from 500 to 800 mg, preferably from 800 to 1000 mg and more preferably from 500 to 1000 mg. The dose of tromethamole (used as a buffering Alkalinizer to enhance the stability of omeprazole in gastric acidity) is between 50-200 mg, preferably from 70- 200 mg and more preferably from 100-350 mg. excipients including specified amounts of xanthan gum, carboxymethylcellulose, saccharin, pro sweet, sodium lauryl sulfate, artificial cherry flavor and Menthol or any acceptable pharmaceutical flavor are incorporated. All ingredients are homogeneously blended and packed in single dose packets.

In embodiment 14 amoxicillin in embodiment 13) is replaced with a mixture of tinidazole and clarithromycin with the rest of active ingredients (omeprazole) and the inactive ingredients (tromethamole and other excipients) are remained unchanged. All ingredients are homogeneously blended and packed in single dose packets.

The packets from embodiment 13) and embodiment 14) are indicated in sequential therapy program for the treatment of gastric and ulcers associated with H. Pylori infections. The packets from embodiment 13) are to be administered twice daily for 5 days, followed by the packets from embodiment 14) to be taken as one packet twice daily for 5 more consecutive days.

In embodiment 15 the multiple unit fixed dosage forms are made in single dose packets comprising omeprazole in a dose from 10 to 60 mg, preferably from 20 to 30 mg and more preferably from 20-40 mg, amoxicillin at a dose from 500 to 800 mg, preferably from 800 to 1000 mg and more preferably from 500 to 1000 mg. The packets also contains tromethamole used as a buffering Alkalinizer at a dose from 50-200 mg, preferably from 70- 200 mg and more preferably from 100-350 mg. Additionally taurolidine/taurultam for bacterial cell cross linking is included in the formula in order to synergies the activity of the amoxicillin and reduce bacterial resistance. Taurolidine is included in doses between 100 - 200 mg, preferably from 200 - 400 mg and more preferably at between 250-300 mg. Specified amounts of excipients including xanthan gum, Carboxymethylcellulose, saccharin, pro sweet, sodium lauryl sulfate, artificial cherry flavor and Menthol or any acceptable pharmaceutical flavor are incorporated. All ingredients are homogeneously blended and packed in single dose packets.

In embodiment 16 amoxicillin in embodiment 15) is replaced with a mixture of tinidazole and clarithromycin with the rest of active ingredients (omeprazole and taurolidine) and the inactive ingredients (tromethamole and other excipients) are remained unchanged. All ingredients are homogeneously blended and packed in single dose packets.

The packets from embodiment 15) and embodiment 16) are indicated in sequential therapy program for the treatment of gastric and duodenal ulcers associated with H. Pylori infections. The packets from embodiment 15) are to be administered twice daily for 5 days, followed by the packets from embodiment (16) to be taken as one packet twice daily for 5 more consecutive days.

In embodiment 17 the taurolidine in embodiment 15) is replaced with zinc amino acid chelate with the rest of active ingredients (omeprazole and amoxicillin) and the inactive ingredients (tromethamole and other excipients) are remained unchanged. All ingredients are homogeneously blended and packed in single dose packets.

In embodiment 18 taurolidine in embodiment 16) is replaced with zinc amino acid chelate with the rest of active ingredients (omeprazole and tinidazole and clarithromycin) and the inactive ingredients (tromethamole and other excipients) are remained unchanged. All ingredients are homogeneously blended and packed in single dose packets.

The packets from embodiment 17) and embodiment 18) are indicated in sequential therapy program for the treatment of gastric and duodenal ulcers associated with H. Pylori infections. The packets from embodiment 17) are to be administered twice daily for 5 days, followed by the packets from embodiment (18) to be taken as one packet twice daily for 5 more consecutive days.

In embodiment 19 the multiple components fixed dosage forms are made in single dose packets are comprising H2 receptor antagonist ranitidine in a dose from 75 - 150 mg, preferably from 100-300 mg, more preferably from 150-300 mg in combination with amoxicillin in a dose from 500 to 800 mg, preferably from 800 to 1000 mg and more preferably from 500 to 1000 mg. Specified amounts of excipients including xanthan gum, Carboxymethylcellulose, saccharin, pro sweet, sodium lauryl sulfate, artificial cherry flavor and Menthol or any acceptable pharmaceutical flavor are incorporated. All ingredients are homogeneously blended and packed in single dose packets.

In embodiment 20 the amoxicillin in embodiment 19) is substituted with a combination containing both tinidazole and clarithromycin with the rest of active ingredients (ranitidine) and the inactive ingredients (the said excipients) are remained unchanged. tinidazole is included in the formulation at a dose of between 100-200 mg, preferably between 300-400 mg and more preferably between 250-500 mg. clarithromycin is included at a dose between 200-300 mg, preferably between 250-400 mg and more preferably from 250-500 mg. All ingredients are homogeneously blended and packed in single dose packets.

The packets from embodiment 19) and embodiment 20) are indicated in sequential therapy program for the treatment of gastric and duodenal ulcers associated with H. Pylori infections. The packets from embodiment (19) are to be administered twice daily for 5 days, followed by the packets from embodiment (20) to be taken as one packet twice daily for 5 more consecutive days.

In embodiment 21 the multiple components fixed dosage forms are made in single dose packets comprising ranitidine (H2 receptor antagonist) in a dose from 75 - 150 mg, preferably from 100-300 mg, more preferably from 150-300 mg in combination with amoxicillin in a dose from 500 to 800 mg, preferably from 800 to 1000 mg and more preferably from 500 to 1000 mg. taurolidine is additionally included in the formula to reducing bacterial resistance against amoxicillin. Taurolidine is included in doses between 100 - 200 mg, preferably from 200 - 400 mg and more preferably at between 250-300 mg. Specified amounts of excipients including xanthan gum, Carboxymethylcellulose, saccharin, pro sweet, sodium lauryl sulfate and Menthol or any acceptable pharmaceutical flavor are incorporated. All ingredients are homogeneously blended and packed in single dose packets.

In embodiment 22 the amoxicillin in embodiment 21) is replaced with a combination of tinidazole and clarithromycin with the rest of the active ingredients (ranitidine and taurolidine) and the inactive ingredients (the said excipients) are remained unchanged. All ingredients are homogeneously blended and packed in single dose packets.

The packets from embodiment 21) and embodiment 22) are indicated in sequential therapy program for the treatment of gastric and duodenal ulcers associated with H. Pylori infections. The packets from embodiment 21) are to be administered twice daily for 5 days, followed by the packets from embodiment 22) to be taken as one packet twice daily for 5 more consecutive days.

In embodiment 23 the taurolidine in embodiment 21) is replaced with zinc amino acid chelate with the rest of the active ingredients (ranitidine and amoxicillin) and the inactive ingredients (the said excipients) are remained unchanged. All ingredients are homogeneously blended and packed in single dose packets.

In embodiment 24 the taurolidine in embodiment 22) is replaced with zinc amino acid chelate with the rest of the active ingredients (ranitidine, tinidazole and clarithromycin) and the inactive ingredients (the said excipients) are remained unchanged. All ingredients are homogeneously blended and packed in single dose packets.

The packets from embodiment (23) and embodiment (24) are indicated in sequential therapy program for the treatment of gastric and duodenal ulcers associated with H. Pylori infections. The packets from embodiment (23) are to be administered twice daily for 5 days, followed by the packets from embodiment (24) to be taken as one packet twice daily for 5 more consecutive days.

In embodiment 25 the multiple components fixed dosage forms are made in the form of pre-mix powders to be constituted with specified volume of purified water to making 100 ml syrup prior to use. Omeprazole is incorporated in a dose from 10 to 60 mg, preferably from 20 to 30 mg and more preferably from 20-40 mg, amoxicillin in a dose from 500 to 800 mg, preferably from 800 to 1000 mg and more preferably from 500 to 1000 mg. The pre mix also contains tromethamole (used as a buffering Alkalinizer to enhance the stability of omeprazole in gastric acidity) in a dose from 50-200 mg, preferably from 70- 200 mg and more preferably from 50-300 mg. Specified amounts of excipients including xanthan gum, Carboxymethylcellulose, saccharin, pro sweet, sodium lauryl sulfate, artificial cherry flavor and Menthol or any acceptable pharmaceutical flavor are incorporated. All ingredients are homogeneously blended and packed in 100 ml semi-opaque plastic or amber glass bottles.

In embodiment 26 the amoxicillin in the multiple components fixed dosage forms in embodiment (25) is substituted with a mixture of tinidazole and clarithromycin with the rest of the active ingredients (omeprazole) and the inactive ingredients (tromethamole and the said excipients) are remained un changed. All ingredients are homogeneously blended and packed in 100 ml semi-opaque plastic or amber glass bottles.

Prior to administration the pre mix ingredients in the bottle from embodiment (25) is reconstituted with specified volume of degassed purified water (supplied in separate plastic sachet) and administered in 5-10 ml dose twice daily for 5 consecutive days. Immediately after finishing the reconstituted ingredient from embodiment (25), the powder mixture in the bottle from embodiment (26) are reconstituted similarly and administered in 5-10 ml dose twice daily for another 5 more consecutive days to complete the treatment course.

In embodiment 27 the multiple components fixed dosage forms are made in the form of pre-mix powders to be constituted with specified volume of purified water to making 100 ml syrup prior to administration. Omeprazole is incorporated in a dose from 10 to 60 mg, preferably from 20 to 30 mg and more preferably from 20-40 mg, amoxicillin at a dose from 500 to 800 mg, preferably from 800 to 1000 mg and more preferably from 500 to 1000 mg. The said pre mix also contains tromethamole (used as a buffering Alkalinizer) at a dose from 50-200 mg, preferably from 70- 200 mg and more preferably from 100-350 mg. Additionally taurolidine/taurultam (cause bacterial cell cross linking) is included in the formula in order to synergies the activity of the amoxicillin and reduce bacterial resistance. Taurolidine is included in doses between 100 - 200 mg, preferably from 200 - 400 mg and more preferably at between 250-300 mg. Specified amounts of excipients including xanthan gum, Carboxymethylcellulose, saccharin, pro sweet, sodium lauryl sulfate, artificial cherry flavor and Menthol or any acceptable pharmaceutical flavor are incorporated. All ingredients are homogeneously blended and packed in 100 ml semi-opaque plastic or amber glass bottles.

In embodiment 28 amoxicillin in embodiment 27) is replaced with a mixture of tinidazole and clarithromycin with the rest of active ingredients omeprazole and taurolidine and the inactive ingredients tromethamole and other excipients are remained unchanged. All ingredients are homogeneously blended and packed in 100 ml semi-opaque plastic or amber glass bottles.

Prior to administration the premix ingredients in the bottle from embodiment 27) is reconstituted with specified volume of degassed purified water supplied in separate plastic sachet and administered in 5-10 ml dose twice daily for 5 consecutive days. Immediately after completing the administration the reconstituted ingredient from embodiment 27), the powder mixture in the bottle from embodiment 28) are reconstituted similarly and administered in 5-10 ml dose twice daily for another 5 more consecutive days to complete the treatment course.

In embodiment 29 the taurolidine in embodiment 27) is replaced with zinc amino acid chelate with the rest of active ingredients omeprazole and amoxicillin and the inactive ingredients tromethamole and other excipients are remained unchanged. All ingredients are homogeneously blended and packed in 100 ml semi-opaque plastic or amber glass bottles.

In embodiment 30 taurolidine in embodiment 28) is replaced with zinc amino acid chelate with the rest of active ingredients omeprazole, tinidazole and clarithromycin) and the inactive ingredients tromethamole and other excipients are remained unchanged. All ingredients are homogeneously blended and packed in 100 ml semi-opaque plastic or amber glass bottles.

Prior to administration the premix ingredients in the bottle from embodiment 29) is reconstituted with specified volume of degassed purified water supplied in separate plastic sachet and administered in 5-10 ml dose twice daily for 5 consecutive days. Immediately after completing the administration the reconstituted ingredient from embodiment 29), the powder mixture in the bottle from embodiment 30) are reconstituted similarly and administered in 5-10 ml dose twice daily for another 5 more consecutive days to complete the treatment course.

In embodiment 31 multiple components fixed dose premix comprising the H2 receptor antagonist ranitidine in a dose from 75 - 150 mg, preferably from 100-300 mg, more preferably from 150-300 mg in combination with amoxicillin in a dose from 500 to 800 mg, preferably from 800 to 1000 mg and more preferably from 500 to 1000 mg is provided. Specified amounts of excipients including xanthan gum, carboxymethylcellulose, saccharin, pro sweet, sodium lauryl sulfate, artificial cherry flavor and menthol or any acceptable pharmaceutical flavor are incorporated. All ingredients are homogeneously blended and packed in 100 ml semi-opaque plastic or amber glass bottles.

In embodiment 32 the amoxicillin in the multi unit fixed dose premix in embodiment 31) is replaced with a mixture of tinidazole and clarithromycin with the rest of active ingredients ranitidine and the inactive ingredients (the said excipients) are remained unchanged. All ingredients are homogeneously blended and packed in 100 ml semi-opaque plastic or amber glass bottles.

Prior to administration the premix ingredients in the bottle from embodiment 31) is reconstituted with specified volume of degassed purified water supplied in separate plastic sachet and administered in 5-10 ml dose twice daily for 5 consecutive days. Immediately after completing the administration of the reconstituted ingredient from embodiment 31), the powder mixture in the bottle from embodiment 32) are reconstituted similarly and administered in 5-10 ml dose twice daily for another 5 more consecutive days to complete the treatment course.

In embodiment 33 multiple components fixed dose premix comprising ranitidine in a dose from 75 - 150 mg, preferably from 100-300 mg, more preferably from 150-300 mg in combination with amoxicillin in a dose from 500 to 800 mg, preferably from 800 to 1000 mg and more preferably from 500 to 1000 mg and with taurolidine in a dose doses between 100 - 200 mg, preferably from 200 - 400 mg and more preferably at between 250-300 mg is provided. Specified amounts of excipients including xanthan gum, Carboxymethylcellulose, saccharin, pro sweet, sodium lauryl sulfate, artificial cherry flavor and Menthol or any acceptable pharmaceutical flavor are incorporated. All ingredients are homogeneously blended and packed in 100 ml semi-opaque plastic or amber glass bottles.

In embodiment 34 the amoxicillin in the multiple components fixed dose premix in embodiment 33) is replaced with a mixture of tinidazole and clarithromycin with the rest of active ingredients ranitidine and taurolidine and the inactive ingredients (the said excipients) are remained unchanged. All ingredients are homogeneously blended and packed in 100 ml semi-opaque plastic or amber glass bottles.

Prior to administration the premix ingredients in the bottle from embodiment 33) is reconstituted with specified volume of degassed purified water (supplied in separate plastic sachet) and administered in 5-10 ml dose twice daily for 5 consecutive days. Immediately after completing the administration of the reconstituted ingredient from embodiment 33), the powder mixture in the bottle from embodiment 34) are reconstituted similarly and administered in 5-10 ml dose twice daily for another 5 more consecutive days to complete the treatment course.

In embodiment 35 the taurolidine in the multiple components fixed dose premix in embodiment 33) is replaced with zinc amino acid chelate with the rest of active ingredients (ranitidine and amoxicillin) and the inactive ingredients (the said excipients) are remained unchanged. All ingredients are homogeneously blended and packed in 100 ml semi-opaque plastic or amber glass bottles.

In embodiment 36 the taurolidine in the multiple components fixed dose premix in embodiment 34) is replaced with zinc amino acid chelate with the rest of active ingredients ranitidine, tinidazole and clarithromycin and the inactive ingredients (the said excipients) are remained unchanged. All ingredients are homogeneously blended and packed in 100 ml semi-opaque plastic or amber glass bottles.

Prior to administration the premix ingredients in the bottle from embodiment 35) is reconstituted with specified volume of degassed purified water supplied in separate plastic sachet and administered in 5-10 ml dose twice daily for 5 consecutive days. Immediately after completing the administration of the reconstituted ingredient from embodiment 35), the powder mixture in the bottle from embodiment 36) are reconstituted similarly and administered in 5-10 ml dose twice daily for another 5 more consecutive days to complete the treatment course.

In embodiment 37 multiple components fixed dose effervescent packets composed from 20 to 40 mg of a PPI, preferably omeprazole plus between 300 to 500 mg taurolidine in combination with specified amount of effervescent salt composed from mixture of sodium bicarbonate and anhydrous citric acid. Tromethamole between 150 -350 mg is included as a buffering agent in order to stabilize the PPI against gastric acidity. Specified amounts of excipients including saccharin, pro sweet, sodium lauryl sulfate, artificial cherry flavor and menthol or any acceptable pharmaceutical flavor are incorporated. All ingredients are homogeneously blended and packed in single dose packets. The packets from embodiment 37) are administered twice daily after being effervesces in about between 50 to 100 ml of purified water.

In embodiment 38 multiple components fixed dose biphasic tablets composed from enteric coated cores housed in instantaneous release outer compressed tablets. The core tablets contain the specified amount of omeprazole plus 1/3^{rd}. the amount of amoxicillin. The outer immediate release housing tablets contain the remainder of amoxicillin with/without the taurolidine.

In embodiment 39 multiple component fixed dose biphasic tablets composed from enteric coated cores containing the PPI plus a mixture composed from 250 mg of tinidazole plus 200 mg of clarithromycin. The immediate release housing tablets contain the remainder specified amounts of tinidazole and clarithromycin mixture with/without the taurolidine.

The biphasic tablets from embodiment 38) and embodiment 39) are indicated in sequential therapy program for the treatment of gastric and duodenal ulcers associated with H. Pylori infections. The tablets from embodiment 38) are to be administered twice daily for 5 days, followed by the compressed tablets from embodiment (39) to be taken as one tablet twice daily for 5 more consecutive days.

In embodiment 40 the taurolidine that may be included in the outer immediate release coat in embodiment 38) is replaced with zinc amino acid chelate with the rest of active ingredients omeprazole and amoxicillin and the inactive ingredients (the said excipients) are remained unchanged.

In embodiment 41 the taurolidine that may be included in the outer immediate release coat in embodiment 39) is replaced with zinc amino acid chelate with the rest of active ingredients omeprazole, tinidazole and clarithromycin and the inactive ingredients (the said excipients) are remained unchanged.

The biphasic tablets from embodiment 40) and embodiment 41) are to be used in sequential therapy program for the treatment of gastric and duodenal ulcers associated with H. Pylori infections. The tablets from embodiment 40) are to be administered twice daily for 5 days, followed by the compressed tablets from embodiment 41) to be taken as one tablet twice daily for 5 more consecutive days.

The foregoing descriptions illustrate the principles, preferred embodiments and methods of preparations and mode of each ingredient in the said formulations. However, the invention should not be construed as being limited to the particular embodiments discussed above. Additional variations of the embodiments discussed above will be appreciated by those skilled in the art.

Therefore, the above-described embodiments should be regarded as illustrative rather than restrictive. Accordingly, it should be appreciated that variations to those embodiments can be made by those skilled in the art without departing from the scope of the invention as defined by the said claims.

The multiple unit said dosage forms including the compressed tablets, biphasic core tablets, packets, premix syrups are all designed to produce immediate therapeutic effect as they release their active ingredients in the stomach rather than other patented enteric coated products that takes longer time to exert their effects as they release their active ingredients in the duodenum. The advantage of the said biphasic core tablets is the instantaneous release of the majority of their antibiotic contents from the housing tablet then release the protected PPI from the enteric coated core together with part of the protected antibiotics, the way by which maintain constant therapeutic effect of the antibiotics. The said preparations in the presented embodiments are suitably administered in sequential way. The said active ingredients are divided into two individual doses. The treatment starts by administering the dose that include amoxicillin, PPI or H2 receptor antagonist with/without the taurolidine twice daily for five consecutive days, followed by administering the doses that include clarithromycin, tinidazole and the PPI together with/without the taurolidine twice daily for another five consecutive days.

The multiple component preparations in the form of premix syrups and/or dispersible packets are suitable for the treatment of children, elderly and patients with swallowing problems. The dosage forms is to be dispersed in purified water before being orally administered or fed through a naso-gastric tube.

The invention is illustrated more in detail in the following examples.

### Example 1: Instant release, stabilized, multiple components tablets comprising omeprazole and amoxicillin (batch size 1,000 tablets).

| g/1000 Tablets | mg/Tablet | Substance |
|---|---|---|
| 20 g | 20 mg | PPI (omeprazole) |
| 1000 g | 1000 mg | amoxicillin |
| 150 g | 150 mg | tromethamole |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Magnesium omeprazole, amoxicillin, tromethamole, microcrystalline cellulose, sodium starch glycolate and Aerosil.RTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared tablets are film coated. The amount of omeprazole in each tablet is 20 mg, the amount of amoxicillin is 1000 mg and the amount of the alkalinizer tromethamole is 150 mg.

### Example 2: Instant release, stabilized, multiple components tablets comprising omeprazole, tinidazole and clarithromycin (batch size 1,000 tablets).

| g/1000 Tablets | mg/Tablet | Substance |
|---|---|---|
| 20 g | 20 mg | PPI (omeprazole) |
| 400 g | 400 mg | clarithromycin |
| 500 g | 500 mg | tinidazole |
| 0150g | 150 mg | tromethamole |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Magnesium omeprazole, clarithromycin, tinidazole, tromethamole, microcrystalline cellulose, sodium starch glycolate and Aerosil.RTM are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared Tablets are film coated. The amount of omeprazole in each tablet is 20 mg, the amount of clarithromycin is 400, the amount of tinidazole is 500 mg and the amount of the alkalinizer tromethamole is 150 mg.

The compressed tablets from embodiment 1) and embodiment 2) are indicated in sequential therapy program for the treatment of gastric and duodenal ulcers associated with H. Pylori infections. The tablets from embodiment 1) are to be administered twice daily for 5 days, followed by the compressed tablets from embodiment 2) to be taken as one tablet twice daily for 5 more consecutive days.

### Example 3: Instant release, stabilized, multiple components tablets comprising omeprazole, amoxicillin and taurolidine (batch size 1,000 tablets).

| g/1000 Tablets | mg/Tablet | Substance |
|---|---|---|
| 20 g | 20 mg | PPI (omeprazole) |
| 1000 g | 1000 mg | amoxicillin |
| 300 g | 300 mg | taurolidine in micro-crystalline form. |
| 0150 g | 150 mg | tromethamole |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Magnesium omeprazole, amoxicillin, taurolidine, tromethamole, microcrystalline cellulose, sodium starch glycolate and Aerosil.RTM are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared tablets are film coated. The amount of omeprazole in each tablet is 20 mg, the amount of amoxicillin is 1000 mg, the amount of taurolidine is 300 mg and the amount of the alkalinizer tromethamole is 150 mg.

### Example 4: Instant release, stabilized, multiple components tablets comprising omeprazole, clarithromycin, tinidazole and taurolidine (batch size 1,000 tablets)

| g/1000 Tablets | mg/Tablet | Substance |
|---|---|---|
| 20 g | 20 mg | PPI (omeprazole) |
| 400 g | 400 mg | clarithromycin |
| 500 g | 500 mg | tinidazole |
| 300 g | 300 mg | taurolidine microcrystalline |
| 0150 g | 150 mg | tromethamole |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Magnesium omeprazole, clarithromycin, tinidazole, taurolidine, tromethamole, microcrystalline cellulose, sodium starch glycolate and Aerosil.RTM are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared Tablets are film coated. The amount of omeprazole in each tablet is 20 mg, the amount of clarithromycin is 400 mg, the amount of tinidazole is 500 mg, the amount of taurolidine is 300 mg and the amount of the alkalinizer tromethamole is 150 mg.

The compressed tablets from embodiment 3) and embodiment 4) are indicated in sequential therapy program for the treatment of gastric and duodenal ulcers associated with H. Pylori infections. The tablets from embodiment 3) are to be administered twice daily for 5 days, followed by the compressed tablets from embodiment 4) to be taken as one tablet twice daily for 5 more consecutive days.

### Example 5: Instant release, stabilized, multiple components tablets comprising omeprazole, amoxicillin and zinc amino acid chelate (batch size 1,000 tablets)

| g/1000 Tablets | mg/Tablet | Substance |
|---|---|---|
| 20 g | 20 mg | PPI (omeprazole) |
| 1000 g | 1000 mg | amoxicillin |
| 75 g | 75 mg | zinc amino acid chelate |
| 0150 g | 150 mg | tromethamole |
| 0050 g | 50 mg | Starch glycolate |
| 0004 9 | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Magnesium omeprazole, amoxicillin, zinc amino acid chelate, tromethamole, microcrystalline cellulose, sodium starch glycolate and Aerosil.RTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared Tablets are film coated. The amount of omeprazole in each tablet is 20 mg, the amount of amoxicillin is 1000 mg, the amount of zinc amino acid chelate is 75 mg and the amount of the alkalinizer tromethamole is 150 mg.

### Example 6: Instant release, stabilized, multiple components tablets comprising omeprazole, clarithromycin, tinidazole and zinc amino acid chelate (batch size 1,000 tablets)

| g/1000 Tablets | mg/Tablet | Substance |
|---|---|---|
| 20 g | 20 mg | PPI (omeprazole) |
| 400 g | 400 mg | clarithromycin |
| 500 g | 500 mg | tinidazole |
| 75 g | 75 mg | zinc amino acid chelate |
| 0150 g | 150 mg | tromethamole |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Magnesium omeprazole, clarithromycin, tinidazole, zinc amino acid chelate, tromethamole, microcrystalline cellulose, sodium starch glycolate and Aerosil.RTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared Tablets are film coated. The amount of omeprazole in each tablet is 20 mg, the amount of clarithromycin is 400 mg, the amount of tinidazole is 500 mg, the amount of zinc amino acid chelate is 75 mg and the amount of the alkalinizer tromethamole is 150 mg.

The tablets from embodiment 5) and embodiment 6) are indicated in sequential therapy program for the treatment of gastric and duodenal ulcers associated with H. Pylori infections. The tablets from embodiment 5) are to be administered twice daily for 5 days, followed by the tablets from embodiment 6) to be taken as one tablet twice daily for 5 more consecutive days.

### Example 7: Instant release, stabilized, multiple components tablets comprising ranitidine (H2 Receptor antagonist) plus amoxicillin (batch size 1,000 tablets)

| g/1000 Tablets | mg/Tablet | Substance |
|---|---|---|
| 150 g | 150 mg | ranitidine |
| 1000 g | 1000 mg | amoxicillin |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Ranitidine, amoxicillin, microcrystalline cellulose, sodium starch glycolate and AerosiLRTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5×19 mm oval punches. The prepared tablets are film coated. The amount of ranitidine is150mg in each tablet, the amount of amoxicillin is 1000 mg.

### Example 8: Instant release, stabilized, multiple components tablets comprising ranitidine, clarithromycin and tinidazole (batch size 1,000 tablets)

| g/1000 Tablets | mg/Tablet | Substance |
|---|---|---|
| 150 g | 150 mg | ranitidine |
| 400 g | 400 mg | clarithromycin |
| 500 g | 500 mg | tinidazole |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Ranitidine, clarithromycin, tinidazole, microcrystalline cellulose, sodium starch glycolate and Aerosil.RTM are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5×19 mm oval punches. The prepared Tablets are film coated. The amount of ranitidine in each tablet is 150 mg, the amount of clarithromycin is 400 mg and the amount of tinidazole is 500 mg.

The compressed tablets from embodiment 7) and embodiment 8) are indicated in sequential therapy program for the treatment of gastric and duodenal ulcers associated with H. Pylori infections. The tablets from embodiment 7) are to be administered twice daily for 5 days, followed by the compressed tablets from embodiment 8) to be taken as one tablet twice daily for 5 more consecutive days.

### Example 9: Instant release, stabilized, multiple components tablets comprising ranitidine, amoxicillin and taurolidine (batch size 1,000 tablets).

| g/1000 Tablets | mg/Tablet | Substance |
|---|---|---|
| 150 g | 150 mg | ranitidine |
| 1000 g | 1000 mg | amoxicillin |
| 300 g | 300 mg | taurolidine |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Ranitidine, amoxicillin, taurolidine, microcrystalline cellulose, sodium starch glycolate and Aerosil.RTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5×19 mm oval punches. The prepared tablets are film coated. The amount of ranitidine in each tablet is 150 mg, the amount of amoxicillin is 1000 mg, and the amount of taurolidine is 300 mg.

### Example 10: Instant release, stabilized, multiple components tablets comprising ranitidine, clarithromycin, tinidazole and taurolidine (batch size 1,000 tablets)

| g/1000 Tablets | mg/Tablet | Substance |
|---|---|---|
| 150 g | 150 mg | ranitidine |
| 400 g | 400 mg | clarithromycin |
| 500 g | 500 mg | tinidazole |
| 300 g | 300 mg | taurolidine microcrystalline |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Ranitidine, clarithromycin, tinidazole, taurolidine, microcrystalline cellulose, sodium starch glycolate and Aerosil.RTM are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5×19 mm oval punches. The prepared Tablets are film coated. The amount of ranitidine in each tablet is 150 mg, the amount of clarithromycin is 400 mg, the amount of tinidazole is 500 mg and the amount of taurolidine is 300 mg.

The tablets from embodiment 9) and embodiment 10) are to be indicated in sequential therapy program for the treatment of gastric and duodenal ulcers associated with H. Pylori infections. The tablets from embodiment 9) are to be administered twice daily for 5 days, followed by the tablets from embodiment 10) to be taken as one tablet twice daily for 5 more consecutive days.

### Example 11: Instant release, stabilized, multiple components tablets comprising ranitidine, amoxicillin and zinc amino acid chelate (batch size 1,000 tablets)

| g/1000 Tablets | mg/Tablet | Substance |
|---|---|---|
| 150 g | 150 mg | ranitidine |
| 1000 g | 1000 mg | amoxicillin |
| 75 g | 75 mg | zinc amino acid chelate |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Ranitidine, amoxicillin, zinc amino acid chelate microcrystalline cellulose, sodium starch glycolate and Aerosil.RTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared Tablets are film coated. The amount of ranitidine in each tablet is 150 mg, the amount of amoxicillin is 1000 mg, and the amount of zinc amino acid chelate is 75 mg.

### Example 12: Instant release, stabilized, multiple components tablets comprising omeprazole, clarithromycin, tinidazole and zinc amino acid chelate (batch size 1,000 tablets)

| g/1000 Tablets | mg/Tablet | Substance |
|---|---|---|
| 150 g | 150 mg | ranitidine |
| 400 g | 400 mg | clarithromycin |
| 500 g | 500 mg | tinidazole |
| 75 g | 75 mg | zinc amino acid chelate |
| 0050 g | 50 mg | Starch glycolate |
| 0004 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |

For preparation sodium lauryl sulfate and povidone K90 are dissolved in purified water to form the granulation liquid. Ranitidine, clarithromycin, tinidazole, and zinc amino acid chelate microcrystalline cellulose, sodium starch glycolate and Aerosil.RTM. are dry-mixed. The granulating liquid is added to the powder mixture and the mass is wet-mixed. The wet mass is dried in a steam-oven. The prepared granulation is milled through sieve 1 mm in oscillating mill equipment. The prepared granules and sodium stearyl fumarate are mixed and compressed into tablets using a rotary tableting machine equipped with 8.5x19 mm oval punches. The prepared Tablets are film coated. The amount of ranitidine in each tablet is 150 mg, the amount of clarithromycin is 400 mg, the amount of tinidazole is 500 mg and the amount of zinc amino acid chelate is 75 mg.

The tablets from embodiment 11) and embodiment 12) are indicated in sequential therapy program for the treatment of gastric and duodenal ulcers associated with H. Pylori infections. The tablets from embodiment 11) are to be administered twice daily for 5 days, followed by the tablets from embodiment 12) to be taken as one tablet twice daily for 5 more consecutive days.

### Example 13: Stabilized, dispersible, multiple components powder/granules in packets comprising omeprazole and amoxicillin (Batch size 1,000 packets).

| g/1000 packets | mg/packet | Substance |
|---|---|---|
| 20 g | 20 mg | PPI (omeprazole) |
| 1000 g | 1000 mg | amoxicillin |
| 150 g | 150 mg | tromethamole |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial Cherry flavor |

For preparation: 1) Magnesium omeprazole, amoxicillin and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of omeprazole in each single packet is 20 mg, the amount of amoxicillin is 1000 mg and the amount of the alkalinizer tromethamole is 150 mg.

### Example 14: Stabilized, dispersible, multiple components powder/granules in packets comprising omeprazole, clarithromycin and tinidazole (batch size 1000 packets).

| g/1000 Packets | mg/Packet | Substance |
|---|---|---|
| 20 g | 20 mg | PPI (omeprazole) |
| 400 g | 400 mg | clarithromycin |
| 500 g | 500 mg | tinidazole |
| 0150 g | 150 mg | tromethamole |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) Magnesium omeprazole, clarithromycin, tinidazole and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of omeprazole in each single packet is 20 mg, the amount of clarithromycin is 400 mg, the amount of tinidazole is 500 mg and the amount of the alkalinizer tromethamole is 150 mg. The packets in example 13) and the packets in example 14) are administered sequentially for a period of 10 days. The packets in example 13) are administered twice daily for 5 consecutive days after being dispersed and stirred in about 30 ml of purified water for immediate administration on an empty stomach. This is followed by the administration of the packets from example 14) twice daily for another 5 consecutive days following the same procedure.

### Example 15: Stabilized, dispersible, multiple components powder/granules in packets comprisinq omeprazole, amoxicillin and taurolidine (batch size 1000 packets).

| g/1000 Packets | mg/ Packet | Substance |
|---|---|---|
| 20 g | 20 mg | PPI (omeprazole) |
| 1000 g | 1000 mg | amoxicillin |
| 300 g | 300 mg | taurolidine |
| 150g | 150 mg | tromethamole |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) Magnesium omeprazole, amoxicillin, taurolidine and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50°C, milled through sieve 1 mm in an oscillating mill and packed in single dose packets in controlled equipment and packed in single dose packets in controlled humidity environment under vacuum. 7) The amount of omeprazole in each single packet is 20 mg, the amount of amoxicillin is 1000 mg, the amount of taurolidine is 300 mg and the amount of the alkalinizer tromethamole is 150 mg.

### Example 16: Stabilized, dispersible, multiple components powder/granules in packets comprising omeprazole clarithromycin, tinidazole and taurolidine (batch size 1000 packets).

| g/1000 Packets | mg/ Packet | Substance |
|---|---|---|
| 20 g | 20 mg | PPI (omeprazole) |
| 400 9 | 400 mg | clarithromycin |
| 500 g | 500 mg | tinidazole |
| 300 g | 300 mg | taurolidine microcrystalline |
| 150 g | 150 mg | tromethamole |
| 35 9 | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) Magnesium omeprazole, clarithromycin, tinidazole, taurolidine and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of omeprazole in each single packet is 20 mg, the amount of clarithromycin is 400 mg, the amount of tinidazole is 500 mg, the amount of taurolidine is 300 mg and the amount of the alkalinizer tromethamole is 150 mg.

The packets in example 15) and the packets in example 16) are administered sequentially for a period of 10 days. The packets in example 15) are administered twice daily for 5 consecutive days after being dispersed and stirred in about 30 ml of purified water for immediate administration on an empty stomach. This is followed by the administration of the packets from example 16) twice daily for another 5 consecutive days following the same procedure.

### Example 17: Stabilized, dispersible, multiple components powder/granules in packets comprising omeprazole, amoxicillin and zinc amino acid chelate (Batch size 1000 packets).

| g/1000 Packets | mg/ Packet | Substance |
|---|---|---|
| 20 g | 20 mg | PPI (omeprazole) |
| 1000 g | 1000 mg | amoxicillin |
| 75 g | 75 mg | zinc amino acid chelate |
| 150 g | 150 mg | tromethamole |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) Magnesium omeprazole, amoxicillin, zinc amino acid chelate and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of omeprazole in each single packet is 20 mg, the amount of amoxicillin is 1000 mg, the amount of taurolidine is 300 mg and the amount of the alkalinizer tromethamole is 150 mg.

### Example 18: Stabilized, dispersible, multiple components powder/granules in packets comprising omeprazole clarithromycin, tinidazole and zinc amino acid chelate (batch size 1000 packets).

| g/1000 Packets | mg/ Packet | Substance |
|---|---|---|
| 20 g | 20 mg | PPI (omeprazole) |
| 400 g | 400 mg | clarithromycin |
| 500 g | 500 mg | tinidazole |
| 75 g | 75 mg | zinc amino acid chelate |
| 150 g | 150 mg | tromethamole |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) Magnesium omeprazole, clarithromycin, tinidazole, zinc amino acid chelate and tromethamole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of omeprazole in each single packet is 20 mg, the amount of clarithromycin is 400 mg, the amount of tinidazole is 500 mg, the amount of taurolidine is 300 mg and the amount of the alkalinizer tromethamole is 150 mg.

The packets in example 17) and the packets in example 18) are administered sequentially for a period of 10 days. The packets in example 17) are administered twice daily for 5 consecutive days after being dispersed and stirred in about 30 ml of purified water for immediate administration on an empty stomach. This is followed by the administration of the packets from example 18) twice daily for another 5 consecutive days following the same procedure.

### Example 19: Stabilized, dispersible, multiple components powder/granules in packets comprising ranitidine and amoxicillin (batch size 1000 packets).

| g/1000 Packets | mg/ Packet | Substance |
|---|---|---|
| 150 g | 150 mg | ranitidine |
| 1000 g | 1000 mg | amoxicillin |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) ranitidine and amoxicillin are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, artificial cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of ranitidine in each single packet is 150 mg and the amount of amoxicillin is 1000 mg.

### Example 20: Stabilized, dispersible, multiple components powder/granules in packets comprising ranitidine, clarithromycin and tinidazole (batch size 1000 packets).

| g/1000 Packets | mg/ Packet | Substance |
|---|---|---|
| 150 g | 150 mg | ranitidine |
| 400 g | 400 mg | clarithromycin |
| 500 g | 500 mg | tinidazole |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) ranitidine, clarithromycin and tinidazole are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, artificial cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of ranitidine in each single packet is 150 mg, the amount of clarithromycin is 400 mg and the amount of tinidazole is 500 mg.

The packets in example 19) and the packets in example 20) are administered sequentially for a period of 10 days. The packets in example 19) are administered twice daily for 5 consecutive days after being dispersed and stirred in about 30 ml of purified water for immediate administration on an empty stomach. This is followed by the administration of the packets from example 20) twice daily for another 5 consecutive days following the same procedure.

### Example 21 Stabilized, dispersible, multiple components powder/granules in packets comprising ranitidine, amoxicillin and taurolidine (batch size 1000 packets).

| g/1000 Packets | mg/ Packet | Substance |
|---|---|---|
| 150 g | 150 mg | ranitidine |
| 1000 g | 1000 mg | amoxicillin |
| 300 g | 300 mg | taurolidine microcrystalline |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) ranitidine, amoxicillin and taurolidine are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, artificial cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of ranitidine in each single packet is 150 mg, the amount of amoxicillin is 1000 mg and the amount of taurolidine is 300 mg.

### Example 22: Stabilized, dispersible, multiple components powder/granules in packets comprising ranitidine, clarithromycin, tinidazole and taurolidine (batch size 1000 packets).

| g/1000 Packets | mg/ Packets | Substance |
|---|---|---|
| 150 g | 150 mg | ranitidine |
| 400 g | 400 mg | clarithromycin |
| 500 g | 500 mg | tinidazole |
| 300 g | 300 mg | taurolidine microcrystalline |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) ranitidine, clarithromycin, tinidazole and taurolidine are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, artificial cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of ranitidine in each single packet is 150 mg, the amount of clarithromycin is 400 mg, the amount of tinidazole is 500 mg and the amount of taurolidine is 300 mg.

The packets in example 21) and the packets in example 22) are administered sequentially for a period of 10 days. The packets in example 21) are administered twice daily for 5 consecutive days after being dispersed and stirred in about 30 ml of purified water for immediate administration on an empty stomach. This is followed by the administration of the packets from example 22) twice daily for another 5 consecutive days following the same procedure.

### Example 23: Stabilized, dispersible, multiple components powder/granules in packets comprising ranitidine, amoxicillin and zinc amino acid chelate (batch size 1000 packets).

| g/1000 Packets | mg/ Packet | Substance |
|---|---|---|
| 150 g | 150 mg | ranitidine |
| 1000 g | 1000 mg | amoxicillin |
| 75 g | 75 mg | zinc amino acid chelate |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) ranitidine, amoxicillin and zinc amino acid chelate are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, artificial cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of ranitidine in each single packet is 300 mg, the amount of amoxicillin is 1000 mg and the amount of zinc amino acid chelate is 75 mg.

### Example 24: Stabilized, dispersible, multiple components powder/granules in packets comprising ranitidine, tinidazole, clarithromycin and zinc amino acid chelate (batch size 1000 packets).

| g/1000 Packets | mg/ Packet | Substance |
|---|---|---|
| 150 g | 150 mg | ranitidine |
| 400 g | 400 mg | clarithromycin |
| 500 g | 500 mg | tinidazole |
| 75 g | 75 mg | zinc amino acid chelate |
| 35 g | 35 mg | Xanthan gum |
| 35 g | 35 mg | Carboxymethylcellulose |
| 3 g | 30 mg | Saccharine Ph. Eur.3 crystalline |
| 3 g | 30 mg | Pro sweet |
| 50 g | 50 mg | Sodium lauryl sulfate |
| 3.2 g | 3.2 mg | Sodium stearyl fumarate |
| 10 g | 10 mg | Menthol crystals |
| Q.S | Q.S | Artificial cherry flavor |

For preparation 1) ranitidine, tinidazole, clarithromycin and zinc amino acid chelate are dry-mixed. 2) Carboxymethylcellulose, saccharine (other artificial or natural sweeteners of approved pharmaceutical application can be used) and Pro sweet are dry mixed. 3) Xanthan gum, sodium lauryl sulfate, artificial cherry flavor and menthol are dry mixed. 4) The mixed powders from steps 1, 2 and 3 are homogeneously blended together. 5) The homogeneous powder mixture from step 4 is packed in single dose packets in controlled humidity environment under vacuum. 6) On the other hand, the homogeneous powder from step 4 is moistened with purified water to obtain fluffy granulations. The fluffy granulation is dried in a steam oven at 50 °C, milled through sieve 1 mm in an oscillating mill and packed in single dose packets in controlled equipment and packed in single dose packets in controlled humidity environment under vacuum. 7) The amount of ranitidine in each single packet is 300 mg, the amount of tinidazole 500 mg, clarithromycin is 400 mg and the amount of zinc amino acid chelate is 75 mg.

The packets in example 23) and the packets in example 24) are administered sequentially for a period of 10 days. The packets in example 23) are administered twice daily for 5 consecutive days after being dispersed and stirred in about 30 ml of purified water for immediate administration on an empty stomach. This is followed by the administration of the packets from example 24) twice daily for another 5 consecutive days following the same procedure.

### Example 25: Stabilized, dispersible, multiple components premix powder/granules in multi dose bottles comprising omeprazole and amoxicillin (Batch size 1000 x 100 ml bottles).

| g/100 ml x 1000 Bottles | mg/ 10 ml | Substance |
|---|---|---|
| 200 g | 20 mg | PPI (omeprazole) |
| 10000 g | 1000 mg | amoxicillin |
| 1500 g | 150 mg | tromethamole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, tromethamole, Menthol and the artificial cherry flavor are dry-mixed. 2) Methylparaben with saccharine are dry-mixed. 3) The mixed powders from step 1) are homogenously mixed with the powder mixture from step 2. 4) Omeprazole and pro sweet with the powder mixture from step (3) are dry-mixed. 5) Xanthan gum with the powder mixture obtained from step (4) is dry-mixed. 6) Sodium citrate with Carboxymethylcellulose is dry-mixed. 7) The mixed powders from steps 4) and 6) are homogeneously blended together. 8) The geometric mixing technique is used to homogenously incorporate by mixing the obtained powder mixture from step 7) with amoxicillin. 9) The homogeneous powder mixture from step 8) is packed in 100 ml semi-opaque hard density plastic bottles with measuring caps under controlled humidity. 10) On the other hand, the homogeneous powder from step 8) is moistened with purified water to obtain fluffy granulations. The fluffy granulation is dried in a steam oven at 50 °C, milled through sieve 1 mm in an oscillating mill and packed as in step 9). 11) The amount of omeprazole in each 10 ml dose after the premix is constituted with purified water and with vigorous shaking 20 mg, the amount of amoxicillin is 1000 mg and the amount of the alkalinizer tromethamole is 150 mg. The bottle content should be administered in a 10 ml dose twice daily for a period of 5 consecutive days.

### Example 26: Stabilized, dispersible, multiple components premix powder/granules in multi dose bottles comprising omeprazole, clarithromycin and tinidazole (Batch size 1000 x 100 ml bottles).

| g/100 ml x 1000 Bottles | mg/ 10 ml | Substance |
|---|---|---|
| 200 g | 20 mg | PPI (omeprazole) |
| 400 g | 400 mg | clarithromycin |
| 5000 g | 500 mg | tinidazole |
| 1500 g | 150 mg | tromethamole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) Probylparaben, sodium lauryl sulfate, tromethamole, Menthol and artificial cherry flavor are dry-mixed. 2) Methylparaben with saccharine are dry-mixed. 3) The mixed powders from step 1) are homogenously mixed with the powder mixture from step 2). 4) Omeprazole and pro sweet with the powder mixture from step (3) are dry-mixed. 5) Xanthan gum with the powder mixture obtained from step (4) is dry-mixed. 6) Sodium citrate with Carboxymethylcellulose is dry-mixed. 7) The mixed powders from steps 4 and 6 are homogeneously blended together. 8) Clarithromycin with tinidazole is dry-mixed. 9) The geometric mixing technique is used to homogenously incorporate by mixing the obtained powder mixture from step 7) with the powder mixture from step 8). 10) The homogeneous powder mixture from step (9) is packed in 100 ml semi-opaque hard density plastic bottles with measuring caps under controlled humidity. 11) On the other hand, the homogeneous powder from step (9) is moistened with purified water to obtain fluffy granulations. The fluffy granulation is dried in a steam oven at 50 °C, milled through sieve 1 mm in an oscillating mill and packed as in step (10).

The premix in example 25) and the premix in example 26) are administered sequentially for a period of 10 days. The premix in example 25) is reconstituted with purified water to a 100 ml volume and administered in 10 ml doses twice daily for 5 consecutive days. This is followed by the administration of the reconstituted premix from example 26) in 10 ml doses twice daily for another 5 consecutive days following the same procedure.

### Example 27: Stabilized, dispersible, multiple components premix powder/granules in multi dose bottles comprising omeprazole, amoxicillin and taurolidine (Batch size 1000 x 100 ml bottles).

| g/100 ml x 1000 Bottles | mg/ 10 ml | Substance |
|---|---|---|
| 200 g | 20 mg | PPI (omeprazole) |
| 10000 g | 1000 mg | amoxicillin |
| 3000 g | 300 mg | taurolidine microcrystalline |
| 1500 g | 150 mg | tromethamole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) Probylparaben, sodium lauryl sulfate, tromethamole, Menthol and artificial cherry flavor are dry-mixed. 2) Methylparaben with saccharine are dry-mixed. 3) The mixed powders from step 1) are homogenously mixed with the powder mixture from step 2). 4) Omeprazole and pro sweet with the powder mixture from step 3) are dry-mixed. 5) Xanthan gum with the powder mixture obtained from step (4) is dry-mixed. 6) Sodium citrate with Carboxymethylcellulose is dry-mixed. 7) The mixed powders from steps 4 and 6 are homogeneously blended together. 8) The amoxicillin powder with taurolidine is dry-mixed. 9) The geometric mixing technique is used to homogenously incorporate by mixing the obtained powder mixture from step (7) with the powder mixture from step (8). 10) The homogeneous powder mixture from step (9) is packed in 100 ml semi-opaque hard density plastic bottles with measuring caps under controlled humidity. 11) On the other hand, the homogeneous powder from step (9) is moistened with purified water to obtain fluffy granulations. The fluffy granulation is dried in a steam oven at 50 °C, milled through sieve 1 mm in an oscillating mill and packed as in step (10). 12) The amount of omeprazole in each 10 ml dose after the premix is constituted with purified water and with vigorous shaking 20 mg, the amount of amoxicillin is 1000 mg, the amount of taurolidine is 300 mg and the amount of the alkalinizer tromethamole is 150 mg. The bottle content should be administered in 10 ml doses twice daily for a period of 5 consecutive days.

### Example 28: Stabilized, dispersible, multiple components premix powder/granules in multi dose bottles comprising omeprazole, clarithromycin, tinidazole and taurolidine (Batch size 1000 x 100 ml bottles).

| g/100 ml x 1000 Bottles | mg/ 10 ml | Substance |
|---|---|---|
| 200 g | 20 mg | PPI (omeprazole) |
| 4000 g | 400 mg | clarithromycin |
| 5000 g | 500 mg | tinidazole |
| 3000 g | 300 mg | taurolidine microcrystalline |
| 1500 g | 150 mg | tromethamole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, tromethamole, Menthol and artificial cherry flavor are dry-mixed. 2) Methylparaben with saccharine are dry-mixed. 3) The mixed powders from step (1) are homogenously mixed with the powder mixture from step 2). 4) Omeprazole and pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 4 and 6 are homogeneously blended together. 8) Clarithromycin with tinidazole and taurolidine are dry-mixed. 9) The geometric mixing technique is used to homogenously incorporate by mixing the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step (9) is moistened with purified water to obtain fluffy granulations. The fluffy granulation is dried in a steam oven at 50 °C, milled through sieve 1 mm in an oscillating mill and packed as in step 10).

The premix in example 27) and the premix in example 28) are administered sequentially for a period of 10 days. The premix in example 27) is reconstituted with purified water to a 100 ml volume and administered in 10 ml doses twice daily for 5 consecutive days. This is followed by the administration of the reconstituted premix from example 28) in 10 ml doses twice daily for another 5 consecutive days following the same procedure.

### Example 29: Stabilized, dispersible, multiple components premix powder/granules in multi dose bottles comprising omeprazole, amoxicillin and zinc amino acid chelate (Batch size 1000 x 100 ml bottles).

| g/100 ml x 1000Bottles | mg/10 ml | Substance |
|---|---|---|
| 200 g | 20 mg | PPI (omeprazole) |
| 10000 g | 1000 mg | amoxicillin |
| 75 g | 75 mg | zinc amino acid chelate |
| 1500 g | 150 mg | tromethamole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) Probylparaben, sodium lauryl sulfate, tromethamole, Menthol and artificial cherry flavor are dry-mixed. 2) Methylparaben with saccharine. 3) The mixed powders from step 1) are homogenously mixed with the powder mixture from step 2). 4) Omeprazole and pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 4) and 6) are homogeneously blended together. 8) The amoxicillin powder with zinc amino acid chelate is dry-mixed. 9) The geometric mixing technique is used to homogenously incorporate by mixing the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step 10). 12) The amount of omeprazole in each 10 ml dose after the premix is constituted with purified water and with vigorous shaking 20 mg, the amount of amoxicillin is 1000 mg, the amount of zinc amino acid chelate is 75 mg and the amount of the alkalinizer tromethamole is 150 mg. The bottle content should be administered in 10 ml doses twice daily for a period of 5 consecutive days.

### Example 30 Stabilized, dispersible, multiple components premix powder/granules in multi dose bottles comprising omeprazole, clarithromycin, tinidazole and zinc amino acid chelate (Batch size 1000 x 100 ml bottles).

| g/100 ml x 1000 Bottles | mg/ 10 ml | Substance |
|---|---|---|
| 200 g | 20 mg | PPI (omeprazole) |
| 4000 g | 400 mg | clarithromycin |
| 5000 g | 500 mg | tinidazole |
| 75 g | 75 mg | zinc amino acid chelate |
| 1500 g | 150 mg | tromethamole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, tromethamole, Menthol and artificial cherry flavor are dry-mixed. 2) Methylparaben with saccharine are dry-mixed. 3) The mixed powders from step 1) are homogenously mixed with the powder mixture from step 2). 4) Omeprazole and pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 4 and 6 are homogeneously blended together. 8) Clarithromycin with tinidazole and zinc amino acid chelate is dry-mixed. 9) The geometric mixing technique is used to homogenously incorporate by mixing the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step (10).

The premix in example 29) and the premix in example 30) are administered sequentially for a period of 10 days. The premix in example 29) is reconstituted with purified water to a 100 ml volume and administered in 10 ml doses twice daily for 5 consecutive days. This is followed by the administration of the reconstituted premix from example 30) in 10 ml doses twice daily for another 5 consecutive days following the same procedure.

### Example 31 Stabilized, dispersible, multiple components premix powder/granules in multi dose bottles comprising ranitidine and amoxicillin (Batch size 1000 x 100 ml bottles).

| g/100 ml x 1000 Bottles | mg/ 10 ml | Substance |
|---|---|---|
| 1500 g | 150 mg | ranitidine |
| 10000 g | 1000 mg | amoxicillin |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, Menthol and artificial cherry flavor are dry-mixed. 2) Methylparaben with saccharine are dry-mixed. 3) The mixed powders from step 1) are homogenously mixed with the powder mixture from step 2). 4) Pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 5) and 6) are homogeneously blended together. 8) Ranitidine with amoxicillin is dry-mixed. 9) The geometric mixing technique is used to homogenously incorporate the obtained powder mixture from step (7) with the powder mixture from step (8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step 9). 12) The amount of ranitidine in each 10 ml dose after the premix is constituted with purified water is 150 mg and the amount of amoxicillin is 1000 mg. The bottle content should be administered in a 10 ml dose twice daily for a period of 5 consecutive days.

### Example 32: Stabilized, dispersible, multiple components premix powder/granules in multi dose bottles comprising ranitidine, clarithromycin and tinidazole (Batch size 1000 x 100 ml bottles).

| g/100 ml x 1000 Bottles | mg/ 10 ml | Substance |
|---|---|---|
| 1500 g | 150 mg | ranitidine |
| 4000 g | 400 mg | clarithromycin |
| 5000 g | 500 mg | tinidazole |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, Menthol and artificial cherry flavor are dry-mixed. 2) Methylparaben with saccharine are dry-mixed. 3) The mixed powders from step 1) are homogenously mixed with the powder mixture from step 2). 4) Omeprazole and pro sweet with the powder mixture from step 3) are dry-mixed. 5) Xanthan gum with the powder mixture obtained from step (4) is dry-mixed. 6) Sodium citrate with Carboxymethylcellulose is dry-mixed. 7) The mixed powders from steps 4 and 6 are homogeneously blended together. 8) Clarithromycin with tinidazole and ranitidine are dry-mixed. 9) The geometric mixing technique is used to homogenously incorporate the obtained powder mixture from step (7) with the powder mixture from step (8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step 10).

The premix in example 31) and the premix in example 32) are administered sequentially for a period of 10 days. The premix in example 31) is reconstituted with purified water to a 100 ml volume and administered in 10 ml doses twice daily for 5 consecutive days. This is followed by the administration of the reconstituted premix from example 32) in 10 ml doses twice daily for another 5 consecutive days following the same procedure.

### Example 33 Stabilized, dispersible, multiple components premix powder/granules in multi dose bottles comprising ranitidine, amoxicillin and taurolidine (Batch size 1000 x 100 ml bottles).

| g/100 ml x 1000 Bottles | mg/ 10 ml | Substance |
|---|---|---|
| 1500 g | 150 mg | ranitidine |
| 10000 g | 1000 mg | amoxicillin |
| 3000 g | 300 mg | taurolidine microcrystalline |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate Menthol and artificial cherry flavor are dry-mixed. 2) Methylparaben with saccharine are dry-mixed. 3) The mixed powders from step 1) are homogenously mixed with the powder mixture from step 2). 4) Pro sweet with the powder mixture from step 3) are dry-mixed. 5) Xanthan gum with the powder mixture obtained from step 4). 6) Sodium citrate with Carboxymethylcellulose is dry-mixed. 7) The mixed powders from steps 5 and 6 are homogeneously blended together. 8) Ranitidine with amoxicillin and taurolidine are dry-mixed. 9) The geometric mixing technique is used to homogenously incorporate the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step 9). 12) The amount of ranitidine in each 10 ml dose after the premix is constituted with purified water is 150 mg, the amount of amoxicillin is 1000 mg and the amount of taurolidine is 300 mg. The bottle content should be administered in 10 ml doses twice daily for a period of 5 consecutive days.

### Example 34: Stabilized, dispersible, multiple components premix powder/granules in multi dose bottles comprising ranitidine, clarithromycin, tinidazole and taurolidine (Batch size 1000 x 100 ml bottles).

| g/100 ml x 1000 Bottles | mg/ 10 ml | Substance |
|---|---|---|
| 1500 g | 150 mg | ranitidine |
| 4000 g | 400 mg | clarithromycin |
| 5000 g | 500 mg | tinidazole |
| 3000 g | 300 mg | taurolidine microcrystalline |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, Menthol and artificial cherry flavor are dry-mixed. 2) Methylparaben with saccharine are dry-mixed. 3) The mixed powders from step 1) are homogenously mixed with the powder mixture from step 2). 4) Omeprazole and pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 4) and 6) are homogeneously blended together. 8) Clarithromycin with tinidazole, taurolidine and ranitidine are dry-mixed. 9) The geometric mixing technique is used to homogenously incorporate the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step 10).

The premix in example 33) and the premix in example 34) are administered sequentially for a period of 10 days. The premix in example 33) is reconstituted with purified water to a 100 ml volume and administered in 10 ml doses twice daily for 5 consecutive days. This is followed by the administration of the reconstituted premix from example 34) in 10 ml doses twice daily for another 5 consecutive days following the same procedure.

### Example 35: Stabilized, dispersible, multiple components premix powder/granules in multi dose bottles comprising ranitidine, amoxicillin and zinc amino acid chelate (Batch size 1000 x 100 ml bottles).

| g/100 ml x 1000 Bottles | mg/10 ml | Substance |
|---|---|---|
| 1500 g | 150 mg | ranitidine |
| 10000 g | 1000 mg | amoxicillin |
| 75 g | 75 mg | zinc amino acid chelate |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, Menthol and artificial cherry flavor are dry-mixed. 2) Methylparaben with saccharine are dry-mixed. 3) The mixed powders from step 1) are homogenously mixed with the powder mixture from step 2). 4) Pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 5) and 6) are homogeneously blended together. 8) Ranitidine with amoxicillin and zinc amino acid chelate is dry-mixed. 9) The geometric mixing technique is used to homogenously incorporate the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step 9). 12) The amount of ranitidine in each 10 ml dose after the premix is constituted with purified water is 150 mg, the amount of amoxicillin is 1000 mg and the amount of zinc amino acid chelate is 75 mg. The bottle content should be administered in 10 ml doses twice daily for a period of 5 consecutive days.

### Example 36: Stabilized, dispersible, multiple components premix powder/granules in multi dose bottles comprising ranitidine, clarithromycin, tinidazole and zinc amino acid chelate (Batch size 1000 x 100 ml bottles).

| g/100 ml x 1000 Bottles | mg/ 10 ml | Substance |
|---|---|---|
| 1500 g | 150 mg | ranitidine |
| 4000 g | 400 mg | clarithromycin |
| 5000 g | 500 mg | tinidazole |
| 75 g | 75 mg | zinc amino acid chelate |
| 350 g | 35 mg | Xanthan gum |
| 350 mg | 35 mg | Sodium citrate anhydrous |
| 350 g | 35 mg | Carboxymethylcellulose |
| 300 g | 30 mg | Saccharine Ph. Eur.3 crystalline |
| 300 g | 30 mg | Pro sweet |
| 0.0665 g | 6.65 mg | Methylparaben |
| 0.0335 g | 3.35 mg | Probylparaben |
| 500 g | 50 mg | Sodium lauryl sulfate |
| 200 g | 2 mg | Menthol crystals |
| Q.S | Q.S | Artificial cherry flavor |

For preparation 1) probylparaben, sodium lauryl sulfate, Menthol and artificial cherry flavor are dry-mixed. 2) Methylparaben with saccharine are dry-mixed. 3) The mixed powders from step 1) are homogenously mixed with the powder mixture from step 2). 4) Omeprazole and pro sweet with the powder mixture from step 3) are dry-mixed. 5) Xanthan gum with the powder mixture obtained from step 4) is dry-mixed. 6) Sodium citrate with Carboxymethylcellulose is dry-mixed. 7) The mixed powders from steps 4) and 6) are homogeneously blended together. 8) Clarithromycin with tinidazole, zinc amino acid chelate and ranitidine are dry-mixed. 9) The geometric mixing technique is used to homogenously incorporate the obtained powder mixture from step 7) with the powder mixture from step 8). 10) The homogeneous powder mixture from step 9) is packed in 100 ml semi-opaque hard density plastic bottles with measuring caps under controlled humidity. 11) On the other hand, the homogeneous powder from step 9) is moistened with purified water to obtain fluffy granulations. The fluffy granulation is dried in a steam oven at 50 °C, milled through sieve 1 mm in an oscillating mill and packed as in step (10).

The premix in example 35) and the premix in example 36) are administered sequentially for a period of 10 days. The premix in example 35) is reconstituted with purified water to a 100 ml volume and administered in 10 ml doses twice daily for 5 consecutive days. This is followed by the administration of the reconstituted premix from example 36) in 10 ml doses twice daily for another 5 consecutive days following the same procedure.

### Example 37: Stabilized, effervescent, multiple components powder in packets comprising PPI and taurolidine (Batch size 1000 Packets)

| g/1000 Packets | mg/ Packet | Substance |
|---|---|---|
| 20 g | 20 mg | omeprazole |
| 300 g | 300 mg | taurolidine |
| 150 g | 150 mg | tromethamole |
| 30 g | 30 mg | Saccharine Ph.Eur.3 crystalline |
| 300 g | 30 mg | Pro sweet |
| 3.2 g | 3.2 mg | Sodium lauryl sulfate |
| 10 g | 10 mg | Menthol crystals |
| Q.S | Q.S | Artificial cherry flavor |
| Q.S | Q.S | Effervescent salt |

For preparation 1) omeprazole, saccharine, pro sweet, menthol, artificial cherry flavor and sodium lauryl sulfate are dry-mixed. 2) Taurolidine and tromethamole are dry-mixed. The powder mixture from step 1) with the powder mixture from step 2) is dry-mixed. 4) The effervescent salt with the powder mixture from step 3) are dry-mixed. 5) All mixing steps are carried-out in controlled humidity environment. 6) The obtained homogenously mixed powders from step 4) are packed in water proofing packets.

For administration one packet is to be effervesce in about 100 ml of purified water and taken twice daily.

### Example 38: Biphasic enteric core tablets comprising mixture of omeprazole plus amoxicillin housed within immediate release compressed coat of amoxicillin (batch size 1,000 tablets).

| g/1000 Tablets | mg/Tablet | Substance |
|---|---|---|
| 20 g | 20 mg | omeprazole |
| 1000 g | 1000 mg | amoxicillin |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |
| | | Solution for separating layer (for 10 kg tablets) |
| 300 g | | Hydroxypropyl methylcellulose |
| 0.003 g | | Hydrogen peroxide (30%) |
| 2701 g | | Purified water |
| | | Solution for enteric coating layer (for 10 kg tablets) |
| 2501 g dispersion (30%) | | Methacrylic acid copolymer |
| 81 g | | Polyethylene glycol 400 |
| 101 g | | Titanium dioxide |
| 1961 ml | | Purified water |

For preparation sodium lauryl sulfate and povidone K90 are dissolved in purified water to form the granulation liquid. Omeprazole plus about 1/3^{rd} of amoxicillin plus 1/3^{rd} of each of microcrystalline cellulose, sodium starch glycolate and Aerosil.RTM. (Aerosil.RTM. is a trademark for fumed silicas available from Degussa) are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and 1/3^{rd} of sodium stearyl fumarate are mixed and compressed into tablets using a rotary tableting machine equipped with a suitable size biconvex or oval punch. The amount of omeprazole in each core tablet is 20 mg; the amount of amoxicillin is about 333.3 mg. The obtained core tablets are covered with a separating layer and an enteric tablet coating layer containing suitable plasticizer to avoid cracking of the enteric coating during housing compression inside the immediate release layer. The immediate release housing tablet is prepared by dry mixing the remainder amount of amoxicillin plus the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator. The prepared granules are mixed with the remainder of sodium stearyl fumarate and compressed around the core tablets by bicota tableting machine using special procedure.

### Example 39: Biphasic enteric core tablets comprising mixture of omeprazole plus half the amount of each tinidazole and clarithromycin housed within immediate release compressed coat comprising mixture of the remainder of tinidazole plus clarithromycin (batch size 1,000 tablets).

| g/1000 Tablets | mg/Tablet | Substance |
|---|---|---|
| 20 g | 20 mg | omeprazole |
| 5000 g | 500 mg | tinidazole |
| 4000 g | 400 mg | clarithromycin |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |
| | | Solution for separating layer (for 10 kg tablets) |
| 300 g | | Hydroxypropyl methylcellulose |
| 0.003 g | | Hydrogen peroxide (30%) |
| 2701 g | | Purified water |
| | | Solution for enteric coating layer (for 10 kg tablets) |
| 2501 g dispersion (30%) | | Methacrylic acid copolymer |
| 81 g | | Polyethylene glycol 400 |
| 101 g | | Titanium dioxide |
| 1961 ml | | Purified water |

For preparation sodium lauryl sulfate and povidone K90 are dissolved in purified water to form the granulation liquid. omeprazole plus a mixture containing 1/2 of the amount of clarithromycin plus 1/2 the amount of tinidazole in addition to 1/^{3rd} of each of microcrystalline cellulose, sodium starch glycolate and Aerosil.RTM. are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and 1/3^{rd} of sodium stearyl fumarate are mixed and compressed into tablets using a rotary tableting machine equipped with a suitable size biconvex or oval punch. The amount of omeprazole in each core tablet is 20 mg, the amount of tinidazole is 250 mg and the amount of clarithromycin is 200 mg. The obtained core tablets are covered with a separating layer and an enteric tablet coating layer containing suitable plasticizer to avoid cracking of the enteric coating during housing compression inside the immediate release layer. The immediate release housing tablet is prepared by dry mixing the remainder amount of tinidazole plus clarithromycin in addition to the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator. The prepared granules are mixed with the remainder of sodium stearyl fumarate and compressed around the core tablets by bicota Tableting machine using special procedure. The amount of tinidazole and clarithromycin in each outer tablet are 250 mg and 200 mg respectively.

### Example 40: Biphasic enteric core tablets comprising mixture of omeprazole plus amoxicillin housed within immediate release compressed coat of amoxicillin plus taurolidine (batch size 1,000 tablets).

| g/1000 Tablets | mg/Tablet | Substance |
|---|---|---|
| 20 g | 20 mg | omeprazole |
| 1000 g | 1000 mg | amoxicillin |
| 300 g | 300 mg | taurolidine |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |
| | | Solution for separating layer (for 10 kg tablets) |
| 300 g | | Hydroxypropyl methylcellulose |
| 0.003 g | | Hydrogen peroxide (30%) |
| 2701 g | | Purified water |
| | | Solution for enteric coating layer (for 10 kg tablets) |
| 2501 g dispersion (30%) | | Methacrylic acid copolymer |
| 81 g | | Polyethylene glycol 400 |
| 101 g | | Titanium dioxide |
| 1961 | | Purified water |

For preparation sodium lauryl sulfate and povidone K90 are dissolved in purified water to form the granulation liquid. Omeprazole plus about 1/3^{rd} of amoxicillin plus 1/3^{rd} of each of microcrystalline cellulose, sodium starch glycolate and Aerosil.RTM. are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and 1/3^{rd} of sodium stearyl fumarate are mixed and compressed into tablets using a rotary tableting machine equipped with a suitable size biconvex or oval punch. The amount of omeprazole in each core tablet is 20 mg; the amount of amoxicillin is about 333.3 mg. The obtained core tablets are covered with a separating layer and an enteric tablet coating layer containing suitable plasticizer to avoid cracking of the enteric coating during housing compression inside the immediate release layer. The immediate release housing tablet is prepared by dry mixing the remainder amount of amoxicillin plus taurolidine plus the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator. The prepared granules are mixed with the remainder of sodium stearyl fumarate and compressed around the core tablets by bicota tableting machine using special procedure. The amount of taurolidine in each outer coat is 300 mg; the amount of amoxicillin is about 666.6 mg.

### Example 42: Biphasic enteric core tablets comprising mixture of omeprazole plus half the amount of each tinidazole and clarithromycin housed within immediate release compressed Tablet comprising mixture of the remainder of tinidazole plus clarithromycin plus taurolidine (batch size 1,000 tablets).

| g/1000 Tablets | mg/Tablet | Substance |
|---|---|---|
| 20 g | 20 mg | omeprazole |
| 5000 g | 500 mg | tinidazole |
| 4000 g | 400 mg | clarithromycin |
| 300 g | 300 mg | taurolidine |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |
| | | Solution for separating layer (for 10 kg tablets) |
| 300 g | | Hydroxypropyl methylcellulose |
| 0.003 g | | Hydrogen peroxide (30%) |
| 2701 g | | Purified water |
| | | Solution for enteric coating layer (for 10 kg tablets) |
| 2501 g dispersion (30%) | | Methacrylic acid copolymer |
| 81 g | | Polyethylene glycol 400 |
| 101 g | | Titanium dioxide |
| 1961 ml | | Purified water |

For preparation sodium lauryl sulfate and povidone K90 are dissolved in purified water to form the granulation liquid. omeprazole plus a mixture containing 1/2 of the amount of clarithromycin plus 1/2 the amount of tinidazole in addition to 1/^{3rd} of each of microcrystalline cellulose, sodium starch glycolate and Aerosil.RTM. are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and 1/3^{rd} of sodium stearyl fumarate are mixed and compressed into tablets using a rotary tableting machine equipped with a suitable size biconvex or oval punch. The amount of omeprazole in each core tablet is 20 mg, the amount of tinidazole is 250 mg and the amount of clarithromycin is 200 mg. The obtained core tablets are covered with a separating layer and an enteric tablet coating layer containing suitable plasticizer to avoid cracking of the enteric coating during housing compression inside the immediate release layer. The immediate release housing tablet is prepared by dry mixing the remainder amount of tinidazole plus clarithromycin plus taurolidine in addition to the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator. The prepared granules are mixed with the remainder of sodium stearyl fumarate and compressed around the core tablets by bicota Tableting machine using special procedure. The amount of taurolidine in each outer tablet is 300 mg; the amount of tinidazole is 250 mg and the amount of clarithromycin is 200 mg.

### Example 43: Biphasic enteric core tablets comprising mixture of omeprazole plus amoxicillin housed within immediate release compressed layer of amoxicillin plus zinc amino acid chelate (batch size 1,000 tablets).

| g/1000 Tablets | mg/Tablet | Substance |
|---|---|---|
| 20 g | 20 mg | omeprazole |
| 1000 g | 1000 mg | amoxicillin |
| 75 g | 75 mg | zinc amino acid chelate |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |
| | | Solution for separating layer (for 10 kg tablets) |
| 300 g | | Hydroxypropyl methylcellulose |
| 0.003 g | | Hydrogen peroxide (30%) |
| 2701 g | | Purified water |
| | | Solution for enteric coating layer (for 10 kg tablets) |
| 2501 g dispersion (30%) | | Methacrylic acid copolymer |
| 81 g | | Polyethylene glycol 400 |
| 101 g | | Titanium dioxide |
| 1961 ml | | Purified water |

For preparation sodium lauryl sulfate and povidone K90 are dissolved in purified water to form the granulation liquid. Omeprazole plus about 1/3^{rd} of amoxicillin plus 1/3^{rd} of each of microcrystalline cellulose, sodium starch glycolate and Aerosil.RTM. are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and 1/3^{rd} of sodium stearyl fumarate are mixed and compressed into tablets using a rotary tableting machine equipped with a suitable size biconvex or oval punch. The amount of omeprazole in each core tablet is 20 mg; the amount of amoxicillin is about 333.3 mg. The obtained core tablets are covered with a separating layer and an enteric tablet coating layer containing suitable plasticizer to avoid cracking of the enteric coating during housing compression inside the immediate release layer. The immediate release housing tablet is prepared by dry mixing the remainder amount of amoxicillin plus zinc amino acid chelate plus the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator. The prepared granules are mixed with the remainder of sodium stearyl fumarate and compressed around the core tablets by bicota tableting machine using special procedure. The amount of zinc amino acid chelate in each outer tablet is 75 mg; the

### Example 44: Biphasic enteric core tablets comprising mixture of omeprazole plus half the amount of each tinidazole and clarithromycin housed within immediate release compressed tablet comprising mixture of the remainder of tinidazole and clarithromycin plus zinc amino acid chelate (batch size 1,000 tablets).

| g/1000 Tablets | mg/Tablet | Substance |
|---|---|---|
| 20 g | 20 mg | omeprazole |
| 4000 g | 400 mg | tinidazole |
| 5000 g | 500 mg | clarithromycin |
| 75 g | 75 mg | zinc amino acid chelate |
| 50 g | 50 mg | Starch glycolate |
| 4 g | 04 mg | Aerosil RTM |
| 3.2 g | 3.2 mg | Sodium lauryl sulfate |
| 150 g | 150 mg | Microcrystalline cellulose |
| 50 g | 50 mg | povidone K90 |
| 18 g | 18 mg | Sodium stearyl fumarate |
| 450 g | 450 mg | Purified water |
| | | Solution for separating layer (for 10 kg tablets) |
| 300 g | | Hydroxypropyl methylcellulose |
| 0.003 g | | Hydrogen peroxide (30%) |
| 2701 g | | Purified water |
| | | Solution for enteric coating layer (for 10 kg tablets) |
| 2501 g dispersion (30%) | | Methacrylic acid copolymer |
| 81 g | | Polyethylene glycol 400 |
| 101 g | | Titanium dioxide |
| 1961 ml | | Purified water |

For preparation sodium lauryl sulfate and povidone K90 are dissolved in purified water to form the granulation liquid. omeprazole plus a mixture containing 1/2 of the amount of clarithromycin plus 1/2 the amount of tinidazole in addition to 1/^{3rd} of each of microcrystalline cellulose, sodium starch glycolate and Aerosil.RTM. are dry-mixed. Specified amount of the granulating liquid is added to the powder mixture and the mass is wet-mixed. The wet mass is dried in a steam-oven. The prepared granulation is milled through sieve 1 mm in an oscillating granulator. The prepared granules and 1/3^{rd} of sodium stearyl fumarate are mixed and compressed into tablets using a rotary tableting machine equipped with a suitable size biconvex or oval punch. The amount of omeprazole in each core tablet is 20 mg, the amount of tinidazole is 200 mg and the amount of clarithromycin is 250 mg. The obtained core tablets are covered with a separating layer and an enteric tablet coating layer containing suitable plasticizer to avoid cracking of the enteric coating during housing compression inside the immediate release layer. The immediate release housing tablet is prepared by dry mixing the remainder amount of tinidazole plus clarithromycin plus zinc amino acid chelate in addition to the remainder of each of microcrystalline cellulose, sodium starch glycolate and Aerosil. The remainder of the granulating liquid is added to the powder mixture and the mass is wet-mixed. The wet mass is dried in oven. The prepared granulation is milled through sieve 1 mm in an oscillating granulator.

The prepared granules are mixed with the remainder of sodium stearyl fumarate and compressed around the core tablets by bicota Tableting machine using special procedure. The amount of zinc amino acid chelate in each outer coat is 300 mg; the amount of tinidazole is 250 mg and the amount of clarithromycin is 200 mg.

Two embodiments of the invention will now be described by way of example with reference to the accompanying drawings, in which
Figure 1 is a schematic cross-section of the monophasic instant release tablet; and
Figure 2 is a schematic cross-section of the biphasic release tablet.

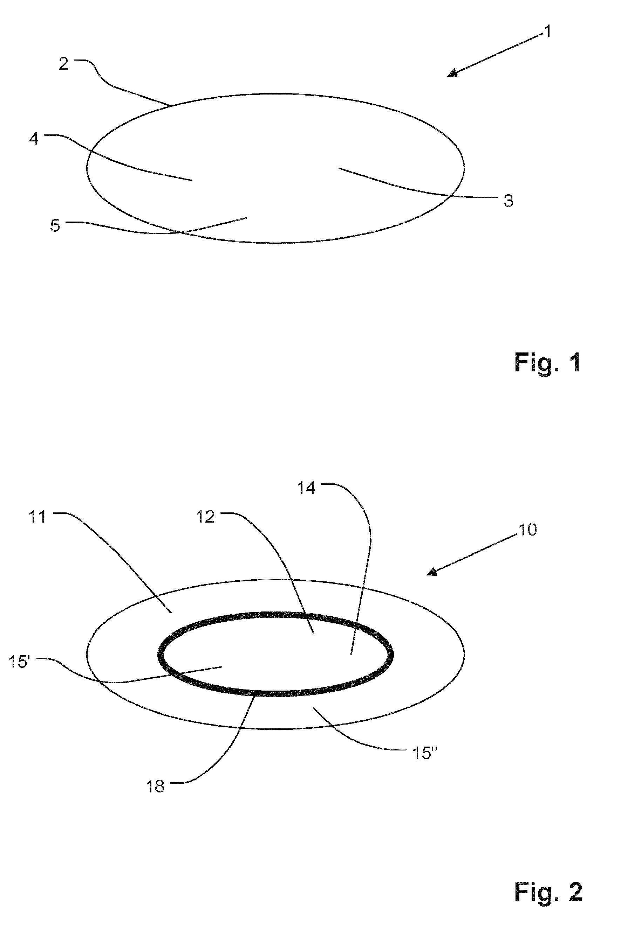

Figure 1 shows a schematic cross-section of instant release tablet 1. The compressed tablet 1 is film coated 2 for an easier swallowing. The tablet 1 comprises only one layer 3 which contains the active ingredients. These are omeprazole 4 in a dose from 20 to 40 mg and amoxicillin 5 in a dose from 500 to 1000 mg. After administration the active ingredients 4, 5 are immediately released in the stomach. To protect omeprazole 3 from the stomach acid the layer 3 also contains tromethamole 6 which acts as a buffering alkalinizer. The tablet comprises 1 also pharmaceutically acceptable excipients (as described above).

Figure 2 is a schematic cross-section of the biphasic release tablet 10. The tablet consists of two layers, an inner, slow release core 12 and an outer, fast release layer 11. Both layers 11, 12 are separated by an enteric coating 18. The inner core contains omeprazole 14 in a dose of 20 mg and amoxicillin 15' in a dose of 333.3 mg. The outer layer contains amoxicillin 15" in a dose of 666.6 mg, so the biphasic release tablet 10 contains an amount of 1000 mg amoxicillin 15 totally. Both layers 11, 12 comprise pharmaceutically acceptable excipients also (as described above).

After administration the outer layer 11 disintegrates in the stomach and 2/3 of the amoxicillin 15" is immediately released. Therefore is has an immediate antibiotic effect. However the inner core 12 remains stable. Only when the inner core 12 has reached the small intestine it disintegrates and the omeprazole 14 and the remaining amount of the amoxicillin 15 is released. Because of the sustained-release of the amoxicillin 15" it has a prolonged antibiotic effect.

The biphasic release tablet 10 has the advantage that it is formulated as a fixed-dose combination which provides a better patient compliance. Another benefit is the biphasic release of the antibiotic.

## Claims

1. An oral pharmaceutical composition for treatment of helicobacter pylori associated diseases comprising an acid blocker together with at least one antibiotic, wherein the formulation is a fixed-dose combination.

2. A pharmaceutical composition according to claim 1, wherein it comprises taurolidine or taurultam or a combination thereof.

3. A pharmaceutical composition according to claim 1 or 2, wherein it comprises zinc amino chelate.

4. A pharmaceutical composition according to one of the preceding claims, wherein the antibiotic is amoxicillin or a combination of tinidazole and clarithromycin.

5. A pharmaceutical composition according to one of the preceding claims, wherein the acid blocker is selected from the group of H2 receptor antagonists, preferably ranitidine.

6. A pharmaceutical composition according to one of the preceding claims, wherein the acid blocker is selected from the group of proton pump inhibitors, preferably omeprazole.

7. A pharmaceutical composition according claim 6, wherein it comprises a suitable pharmaceutical alkanizer, preferably tromethamole.

8. A pharmaceutical composition according to one of the preceding claims, wherein it is an instant release formulation.

9. A pharmaceutical composition according to one of the preceding claims, wherein it is formulated as tablets, capsules, effervescent granules or powder, effervescent tablets, liquids, dispersible and/or soluble tablets, dispersible powder, suspensions, sachets, premix syrups, jellies or glycerites, preferably as compressed tablets.

10. A pharmaceutical composition according to one of the claims 1 to 6, wherein it is formulated as a biphasic release form, preferably as a biphasic tablet with an inner slow release layer and an outer fast release layer.

11. A pharmaceutical composition according to claims 10, wherein the inner layer is enteric coated and comprises the proton pump inhibitor, preferably omeprazole, and the outer layer comprises the antibiotic or the combination of antibiotics, whereby the outer layer is preferably formulated as a compressed tablet.

12. A pharmaceutical composition according to claim 10 to 11, wherein the slow release layer comprises an amount of the antibiotic and the fast release layer comprises the remaining amount of the antibiotic or the remaining amount of the combined antibiotics.

13. A pharmaceutical composition according to claims 10 to 12, wherein the antibiotic is amoxicillin.

14. A pharmaceutical composition according to claims 10 to 13, wherein the combination of antibiotics comprises tinidazole and clarithromycin.

15. A pharmaceutical composition according to claims 10 to 14, wherein the outer layer also comprises taurolidine.
